# EUROPÄISCHE PATENTSCHRIFT

(11) **EP 2 883 422 B1**
(45) Veröffentlichungstag und Bekanntmachung des Hinweises auf die Patenterteilung: **07.03.2018**
(21) Anmeldenummer: 13737142.3
(22) Anmeldetag: 11.07.2013
(51) Int. Cl.: H05B 33/20, C07D 471/16, H01L 51/00, H01L 51/50, C07C 13/72, H01L 51/52

(54) **MATERIALIEN FÜR ORGANISCHE ELEKTROLUMINESZENZVORRICHTUNGEN**
MATERIALS FOR ORGANIC ELECTROLUMINESCENCE DEVICES
MATÉRIAUX POUR DISPOSITIFS ORGANIQUES À ÉLECTROLUMINESCENCE

(30) Priorität: 10.08.2012 EP 12005829
(43) Veröffentlichungstag der Anmeldung: 17.06.2015
(73) Patentinhaber: Merck Patent GmbH, 64293 Darmstadt (DE)
(72) Erfinder: MONTENEGRO, Elvira, 69469 Weinheim (DE); PARHAM, Amir Hossain, 60486 Frankfurt am Main (DE); JATSCH, Anja, 60489 Frankfurt am Main (DE); PFLUMM, Christof, 64291 Darmstadt (DE); KROEBER, Jonas Valentin, 60311 Frankfurt am Main (DE); EBERLE, Thomas, 76829 Landau (DE)
(86) Internationale Anmeldenummer: PCT/EP2013/002057
(87) Internationale Veröffentlichungsnummer: WO 2014/023388

(56) Entgegenhaltungen:
- DE-A1- 4 446 818
- JP-A- 2010 027 681
- US-A1- 2012 126 179

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen enthaltend diese Materialien.

Der Aufbau organischer Elektrolumineszenzvorrichtungen (OLEDs), in denen organische Halbleiter als funktionelle Materialien eingesetzt werden, ist beispielsweise in US 4,539,507, US 5,151,629, EP 0676461 und WO 98/27136 beschrieben. Als emittierende Materialien werden hierbei zunehmend metallorganische Komplexe eingesetzt, die Phosphoreszenz statt Fluoreszenz zeigen (M. A. Baldo et al., Appl. Phys. Lett. 1999, 75, 4-6).

Gemäß dem Stand der Technik werden als Matrixmaterialien für phosphoreszierende Verbindungen sowie als Elektronentransportmaterialien häufig heteroaromatische Verbindungen eingesestzt, wie zum Beispiel Triazinderivate oder Benzimidazolderivate. Als Matrixmaterialien für phosphoreszierende Verbindungen eignen sich auch Carbazolderivate. Bekannt für diese Funktion sind beispielsweise Spirobifluorenderivate, welche in 2-Position mit Triazingruppen substituiert sind, wie in WO 2010/015306 und WO 2010/072300 offenbart. Weiterhin bekannt sind Spirobifluorenderivate, welche in 4,4'-Position mit zwei Triazingruppen substituiert sind. JP 2010-027681 offenbart Bis(N-carbazolyl)spirobifluoren-Derivative, wobei die Carbazolylgruppen jeweils über ein N-Atom in verschiedenen Positionen des Spirobi fluorens, unter anderem in der 4-Position, gebunden sind. Bei diesen Verbindungen gibt es sowohl bei fluoreszierenden wie auch bei phosphoreszierenden OLEDs weiterhin Verbesserungsbedarf, insbesondere hinsichtlich Effizienz, Lebensdauer und Betriebsspannung bei Verwendung in einer organischen Elektrolumineszenzvorrichtung.

Aufgabe der vorliegenden Erfindung ist die Bereitstellung von Verbindungen, welche sich für den Einsatz in einer fluoreszierenden oder phosphoreszierenden OLED, insbesondere einer phosphoreszierenden OLED, eignen, beispielsweise als Elektronentransportmaterial in einer Elektronentransport- bzw. Lochblockierschicht oder als Matrixmaterial in einer emittierenden Schicht.

Überraschend wurde gefunden, dass die unten beschriebenen Verbindungen diese Aufgabe lösen und zu deutlichen Verbesserungen der organischen Elektrolumineszenzvorrichtung führen, insbesondere hinsichtlich der Lebensdauer, der Effizienz und der Betriebsspannung. Dies gilt für phosphoreszierende und fluoreszierende Elektrolumineszenzvorrichtungen, vor allem bei Einsatz der erfindungsgemäßen Verbindungen als Elektronentransportmaterial oder als Matrixmaterial. Die Materialien weisen im Allgemeinen eine hohe thermische Stabilität auf und lassen sich daher unzersetzt und rückstandsfrei sublimieren. Diese Materialien sowie elektronische Vorrichtungen, welche derartige Verbindungen enthalten, sind daher der Gegenstand der vorliegenden Erfindung.

Gegenstand der vorliegenden Erfindung ist daher eine Verbindung gemäß der folgenden Formel (1) oder (2), 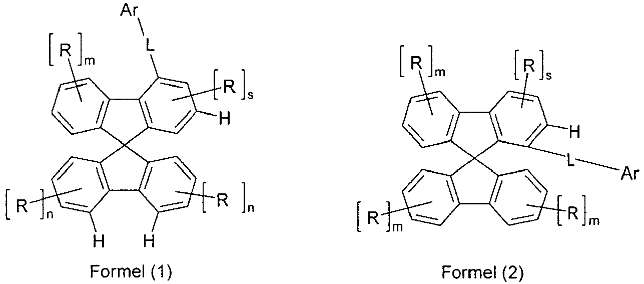 wobei für die verwendeten Symbole und Indizes gilt:
- Ar: ist ein heteroaromatisches Ringsystem mit 5 bis 40 aromatischen Ringatomen, welches über ein Kohlenstoffatom an L gebunden ist, wenn L für eine Einfachbindung steht, und welches über ein Kohlenstoffatom oder ein Stickstoffatom an L gebunden ist, wenn L ungleich einer Einfachbindung ist, und welches durch einen oder mehrere Reste R¹ substituiert sein kann; oder Ar ist ein aromatisches Ringsystem mit 6 bis 40 aromatischen Ringatomen, welches durch einen oder mehrere Reste R¹ substituiert sein kann, wenn L für C(=O) steht;
- L: ist eine Einfachbindung, C(=O) oder ein aromatisches Ringsystem mit 5 bis 24 aromatischen Ringatomen, welches durch einen oder mehrere Reste R substituiert sein kann;
- R, R¹: ist bei jedem Auftreten gleich oder verschieden ausgewählt aus der Gruppe bestehend aus H, D, F, Cl, Br, I, CN, Si(R²)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils mit einem oder mehreren Resten R² substituiert sein kann, wobei jeweils eine oder mehrere nicht-benachbarte CH₂-Gruppen durch Si(R²)₂, C=NR², P(=O)(R²), SO, SO₂, NR², O, S oder CONR² ersetzt sein können und wobei ein oder mehrere H-Atome durch D, F, Cl, Br oder I ersetzt sein können, einem aromatischen oder heteroaromatischen Ringsystem mit 6 bis 40 C-Atomen, das jeweils mit einem oder mehreren Resten R² substituiert sein kann, einer Aryloxygruppe mit 5 bis 60 aromatischen Ringatomen, die mit einem oder mehreren Resten R² substituiert sein kann, oder einer Aralkylgruppe mit 5 bis 60 aromatischen Ringatomen, die jeweils mit einem oder mehreren Resten R² substituiert sein kann, wobei optional zwei oder mehr benachbarte Substituenten R bzw. R¹ ein mono- oder polycyclisches, aliphatisches Ring-system bilden können, das mit einem oder mehreren Resten R² substituiert sein kann;
- R²: ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R² miteinander ein mono- oder polycyclisches, aliphatisches, Ringsystem bilden können;
- s: ist 0, 1 oder 2;
- m: ist bei jedem Auftreten gleich oder verschieden 0, 1, 2, 3 oder 4;
- n: ist bei jedem Auftreten gleich oder verschieden 0, 1, 2 oder 3.

Eine Arylgruppe im Sinne dieser Erfindung enthält 6 bis 24 C-Atome; eine Heteroarylgruppe im Sinne dieser Erfindung enthält 2 bis 24 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl oder Quaterphenyl, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

In einer bevorzugten Ausführungsform der Erfindung sind die Verbindungen der Formel (1) ausgewählt aus den Verbindungen der folgenden Formel (1a) und die Verbindungen der Formel (2) aus den Verbindungen der folgenden Formel (2a), 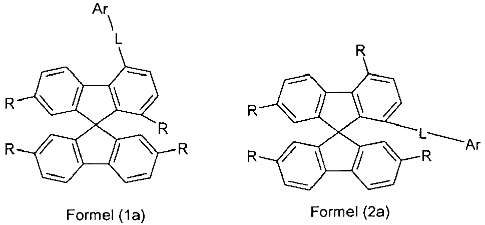 wobei die verwendeten Symbole die oben genannten Bedeutungen aufweisen.

Besonders bevorzugt sind die Verbindungen der folgenden Formeln (1b) und (2b), 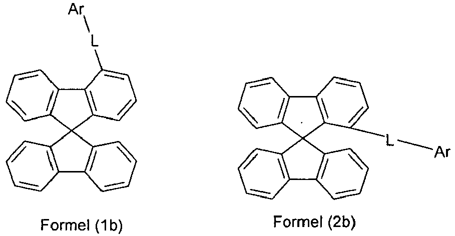 wobei die verwendeten Symbole die oben genannten Bedeutungen aufweisen.

Ganz besonders bevorzugt sind die Verbindungen der Formel (1) bzw. (1a) bzw. (1b), in denen die Gruppe -L-Ar in der 4-Position des Spirobifluorens gebunden ist.

In einer bevorzugten Ausführungsform der Erfindung ist L eine Einfachbindung, C(=O) oder ein aromatisches Ringsystem mit 6 bis 12 aromatischen Ringatomen, welches mit einem oder mehreren Resten R substituiert sein kann. Besonders bevorzugt ist L eine Einfachbindung oder eine ortho-, meta- oder para-verknüpfte Phenylengruppe, welche mit einem oder mehreren Resten R substituiert sein kann, bevorzugt aber unsubstituiert ist.

In einer weiteren bevorzugten Ausführungsform der Erfindung ist Ar ein heteroaromatisches Ringsystem mit 5 bis 24 aromatischen Ringatomen, insbesondere mit 5 bis 13 aromatischen Ringatomen, das jeweils durch einen oder mehrere Reste R¹ substituiert sein kann. Dabei ist die Gruppe Ar über ein Kohlenstoffatom an L gebunden ist, wenn L für eine Einfachbindung steht. Weiterhin kann sie auch über ein Stickstoffatom an L gebunden sein, wenn L ungleich einer Einfachbindung ist, z. B. eine über das Stickstoffatom gebundene Carbazolgruppe, Indolocarbazolgruppe oder Indenocarbazolgruppe. Weiterhin bevorzugt ist Ar ein aromatisches Ringsystem mit 6 bis 24 aromatischen Ringatomen, das durch einen oder mehrere Reste R¹ substituiert sein kann, wenn L für C(=O) steht.

Besonders bevorzugte Gruppen Ar sind ausgewählt aus der Gruppe bestehend aus Triazin, Pyrimidin, Pyrazin, Pyridazin, Pyridin, Pyrazol, Imidazol, Oxazol, Oxadiazol, Thiazol, Benzimidazol, Benzofuran, Benzothiophen, Indol, Dibenzofuran, Dibenzothiophen, Carbazol, Indenocarbazol und Indolocarbazol, wobei diese Gruppen jeweils durch einen oder mehrere Reste R¹ substituiert sein können.

In einer besonders bevorzugten Ausführungsform der Erfindung ist die Gruppe Ar ausgewählt aus den Strukturen der folgenden Formeln (Ar-1) bis (Ar-24), 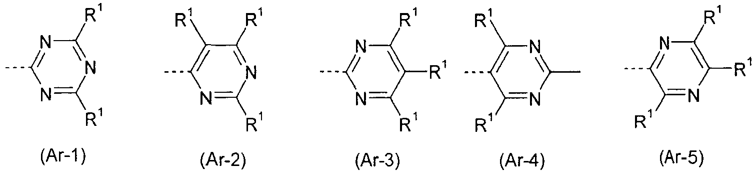 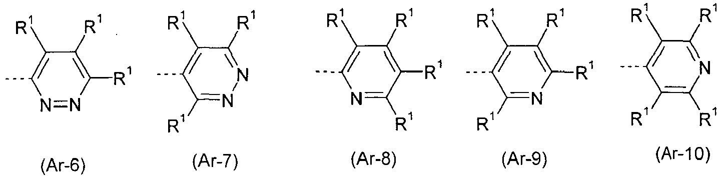 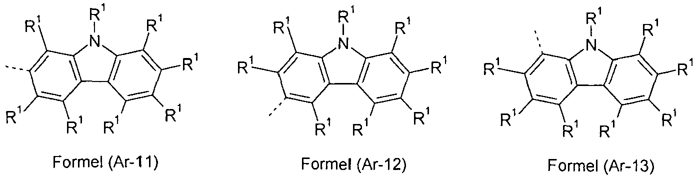 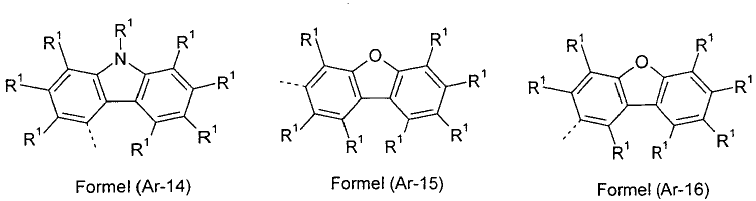 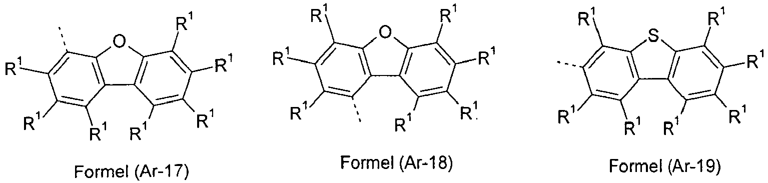 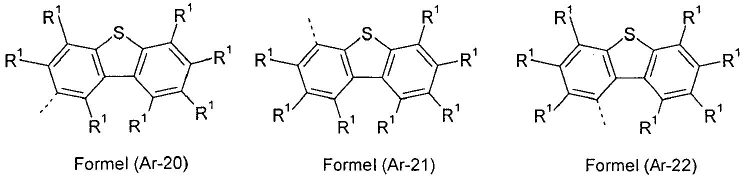 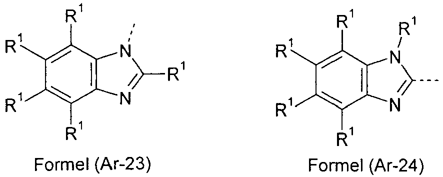 wobei die gestrichelte Bindung die Bindung an L andeutet und R¹ die oben genannten Bedeutungen aufweist.

Weiterhin steht der Rest R¹, der in den Gruppen (Ar-11) bis (Ar-14) und (Ar-24) an das Stickstoffatom gebunden ist, bevorzugt für eine Phenylgruppe, die mit einem oder mehreren Resten R² substituiert sein kann.

Besonders bevorzugt ist Ar eine Triazingruppe, also eine Gruppe der oben genannten Formel (Ar-1).

Wenn L für C(=O) steht, steht Ar weiterhin bevorzugt für ein aromatisches Ringsystem, ausgewählt aus der Gruppe bestehend aus Phenyl, Biphenyl, ortho-, meta- oder para-Terphenyl, ortho-, meta-, para- oder verzweigtes Quaterphenyl, 1-, 2-, 3- oder 4-Fluorenyl oder 1-, 2-, 3- oder 4-Spirobifluorenyl, welches jeweils mit einem oder mehreren Resten R¹ substituiert sein kann.

In einer bevorzugten Ausführungsform der Erfindung ist R bzw. R¹ gleich oder verschieden bei jedem Auftreten ausgewählt aus der Gruppe bestehend aus H, D, F, CN, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch F ersetzt sein können, einem aromatischen oder heteroaromatischen Ringsystem mit 6 bis 24 aromatischen Ringatomen, das jeweils mit einem oder mehreren Resten R² substituiert sein kann.

In einer besonders bevorzugten Ausführungsform der Erfindung ist R bzw. R¹ bei jedem Auftreten gleich oder verschieden ausgewählt aus der Gruppe bestehend aus H, F, einer geradkettigen Alkylgruppe mit 1 bis 5 C-Atomen oder einer verzweigten oder cyclischen Alkylgruppe mit 3 bis 6 C-Atomen, einem aromatischen oder heteroaromatischen Ringsystem mit 5 bis 18 aromatischen Ringatomen, das jeweils mit einem oder mehreren Resten R² substituiert sein kann.

Dabei weisen die Reste R, R¹ und R² bevorzugt keine kondensierten Aryl- oder Heteroarylgruppen auf, in denen mehr als zwei aromatische bzw. heteroaromatische Sechsringe direkt aneinander ankondensiert sind, also beispielsweise keine Anthracen- oder Pyrengruppen. Besonders bevorzugt weisen die Reste R, R¹ und R² überhaupt keine kondensierten Aryl- oder Heteroarylgruppen auf, in denen aromatische bzw. heteroaromatische Sechsringe direkt aneinander ankondensiert sind, also auch beispielsweise keine Naphthalingruppen.

Dabei haben für Verbindungen, die durch Vakuumverdampfung verarbeitet werden, die Alkylgruppen bevorzugt nicht mehr als vier C-Atome, besonders bevorzugt nicht mehr als 1 C-Atom. Für Verbindungen, die aus Lösung verarbeitet werden, eignen sich auch Verbindungen, die mit linearen, verzweigten oder cyclischen Alkylgruppen mit bis zu 10 C-Atomen substituiert sind oder die mit Oligoarylengruppen, beispielsweise ortho-, meta-, para- oder verzweigte Terphenyl- oder Quaterphenylgruppen, substituiert sind.

In einer bevorzugten Ausführungsform der Erfindung ist R² gleich oder verschieden bei jedem Auftreten ausgewählt aus der Gruppe bestehend aus H, einer geradkettigen Alkylgruppe mit 1 bis 10 C-Atomen oder einer verzweigten oder cyclischen Alkylgruppe mit 3 bis 10 C-Atomen oder einem aromatischen Ringsystem mit 6 bis 24 C-Atomen. Besonders bevorzugt ist R² gleich oder verschieden bei jedem Auftreten H oder eine Methylgruppe, ganz besonders bevorzugt H.

Besonders bevorzugt sind Verbindungen der Formeln (1), (1a), (1b) bzw. (2), (2a) und (2b), in denen die oben genannten bevorzugten Ausführungsformen gleichzeitig auftreten. Besonders bevorzugt sind daher Verbindungen, für die gilt:
- L: ist eine Einfachbindung, C(=O) oder ein aromatisches Ringsystem mit 6 bis 12 aromatischen Ringatomen, welches mit einem oder mehreren Resten R substituiert sein kann;
- Ar: ist ein heteroaromatisches Ringsystem mit 5 bis 13 aromatischen Ringatomen, das durch einen oder mehrere Reste R¹ substituiert sein kann, wobei Ar über ein Kohlenstoffatom an L gebunden ist, wenn L für eine Einfachbindung stehtl, oder über ein Kohlenstoff- oder Stickstoffatom an L gebunden ist, wenn L ungleich einer Einfachbindung ist; oder ist ein aromatisches Ringsystem mit 6 bis 24 aromatischen Ringatomen, das durch einen oder mehrere Reste R¹ substituiert sein kann, wenn L für C(=O) steht;
- R, R¹: ist gleich oder verschieden bei jedem Auftreten ausgewählt aus der Gruppe bestehend aus H, D, F, CN, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch F ersetzt sein können, einem aromatischen oder heteroaromatischen Ringsystem mit 6 bis 24 aromatischen Ringatomen, das jeweils mit einem oder mehreren Resten R² substituiert sein kann.
Ganz besonders bevorzugt sind Verbindungen der Formeln (1), (1a), (1b) bzw. (2), (2a) und (2b), für die gilt:
- L: ist eine Einfachbindung oder eine ortho-, meta- oder para-verknüpfte Phenylengruppe, welche mit einem oder mehreren Resten R substituiert sein kann, bevorzugt aber unsubstituiert ist;
- Ar: ist ausgewählt aus der Gruppe bestehend aus Triazin, Pyrimidin, Pyrazin, Pyridazin, Pyridin, Pyrazol, Imidazol, Oxazol, Oxadiazol, Thiazol, Benzimidazol, Benzofuran, Benzothiophen, Indol, Dibenzofuran, Dibenzothiophen, Carbazol, Indenocarbazol und Indolocarbazol, wobei diese Gruppen jeweils durch einen oder mehrere Reste R¹ substituiert sein können, bevorzugt Gruppen der Formeln (Ar-1) bis (Ar-22);
- R, R¹: ist bei jedem Auftreten gleich oder verschieden ausgewählt aus der Gruppe bestehend aus H, F, einer geradkettigen Alkylgruppe mit 1 bis 5 C-Atomen oder einer verzweigten oder cyclischen Alkylgruppe mit 3 bis 6 C-Atomen, einem aromatischen oder heteroaromatischen Ringsystem mit 5 bis 18 aromatischen Ringatomen, das jeweils mit einem oder mehreren Resten R² substituiert sein kann.
Beispiele für geeignete erfindungsgemäße Verbindungen sind die in der folgenden Tabelle aufgeführten Verbindungen:

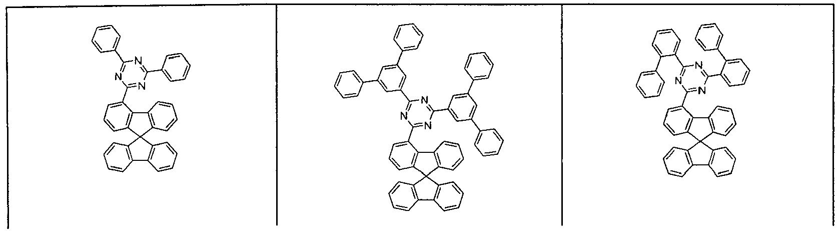

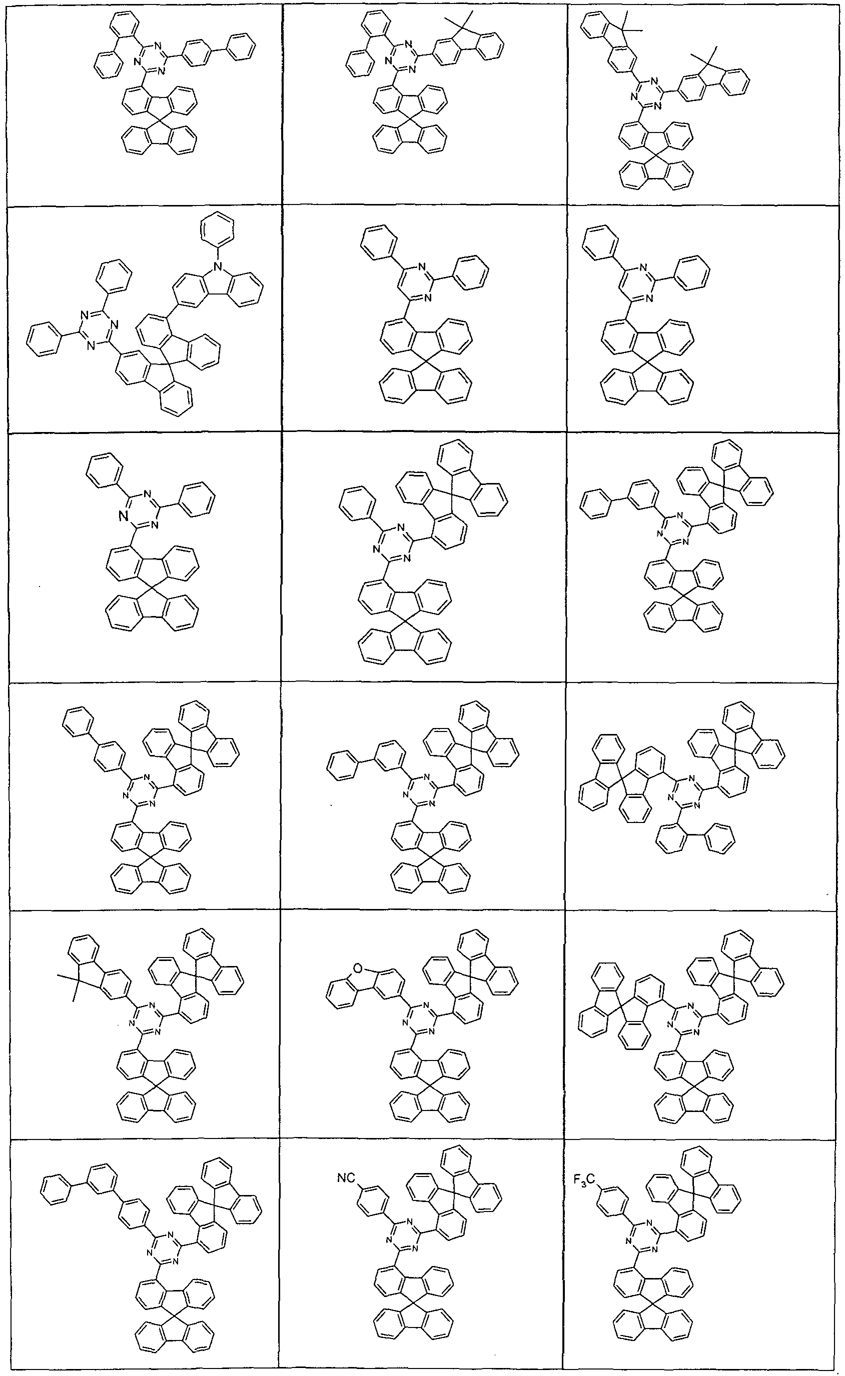

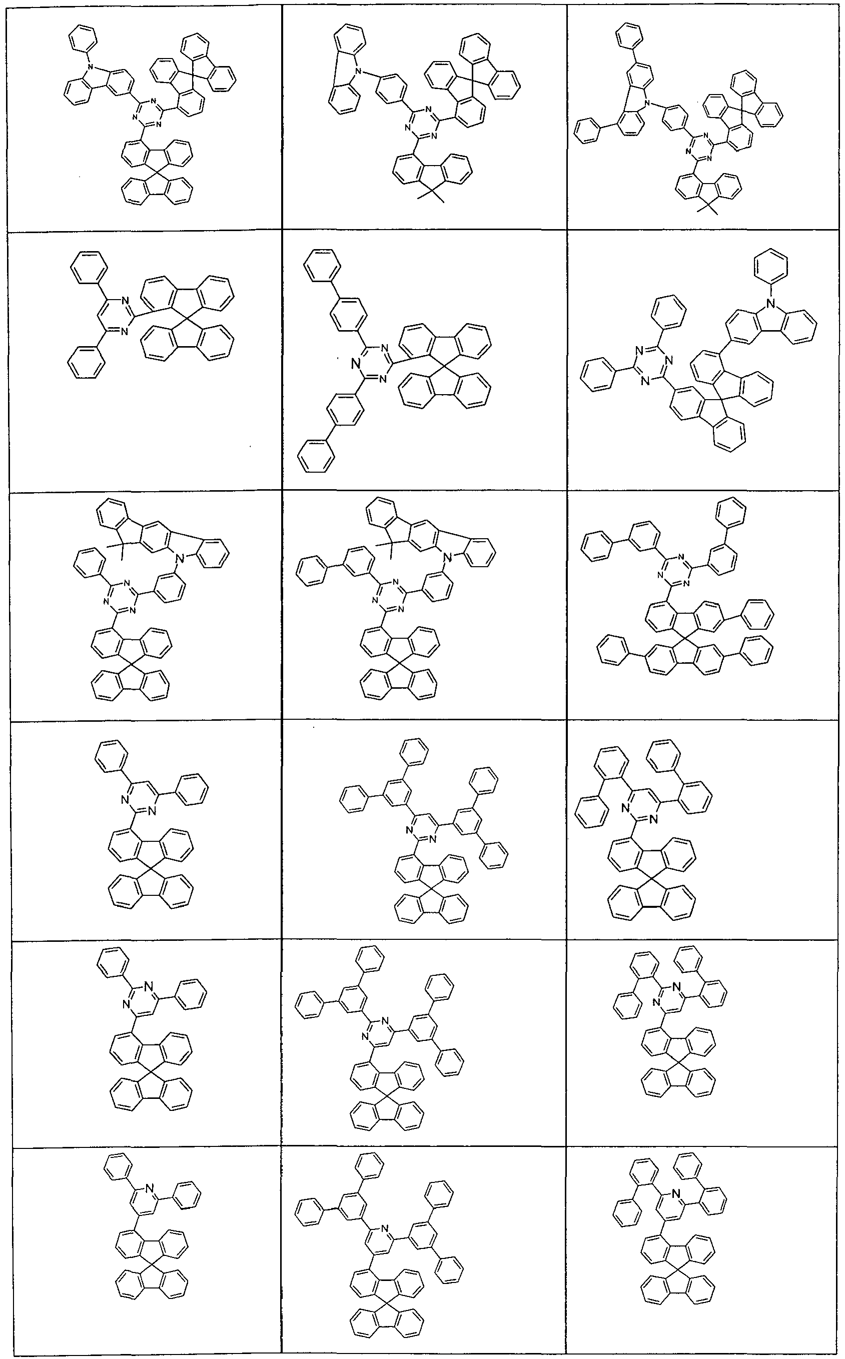

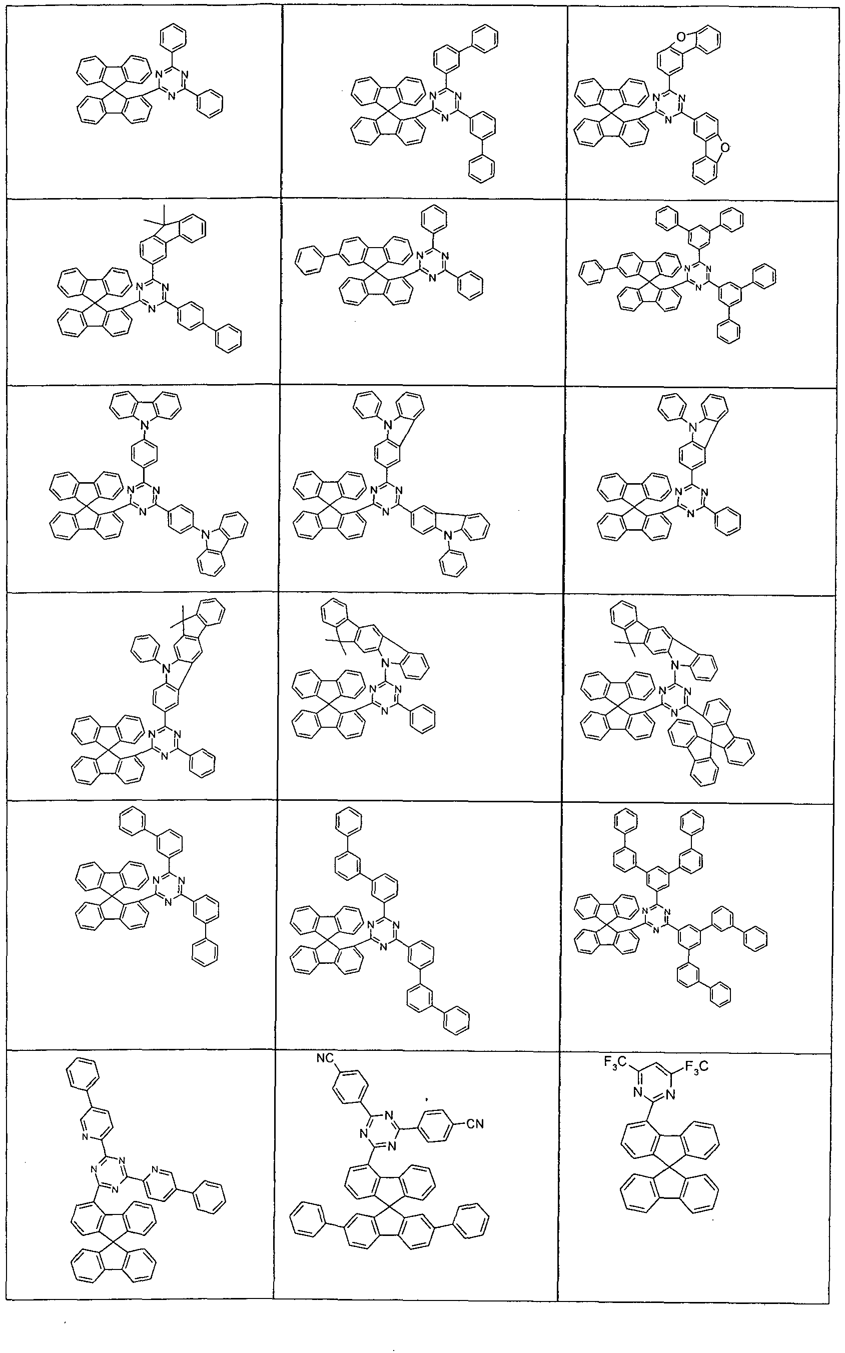

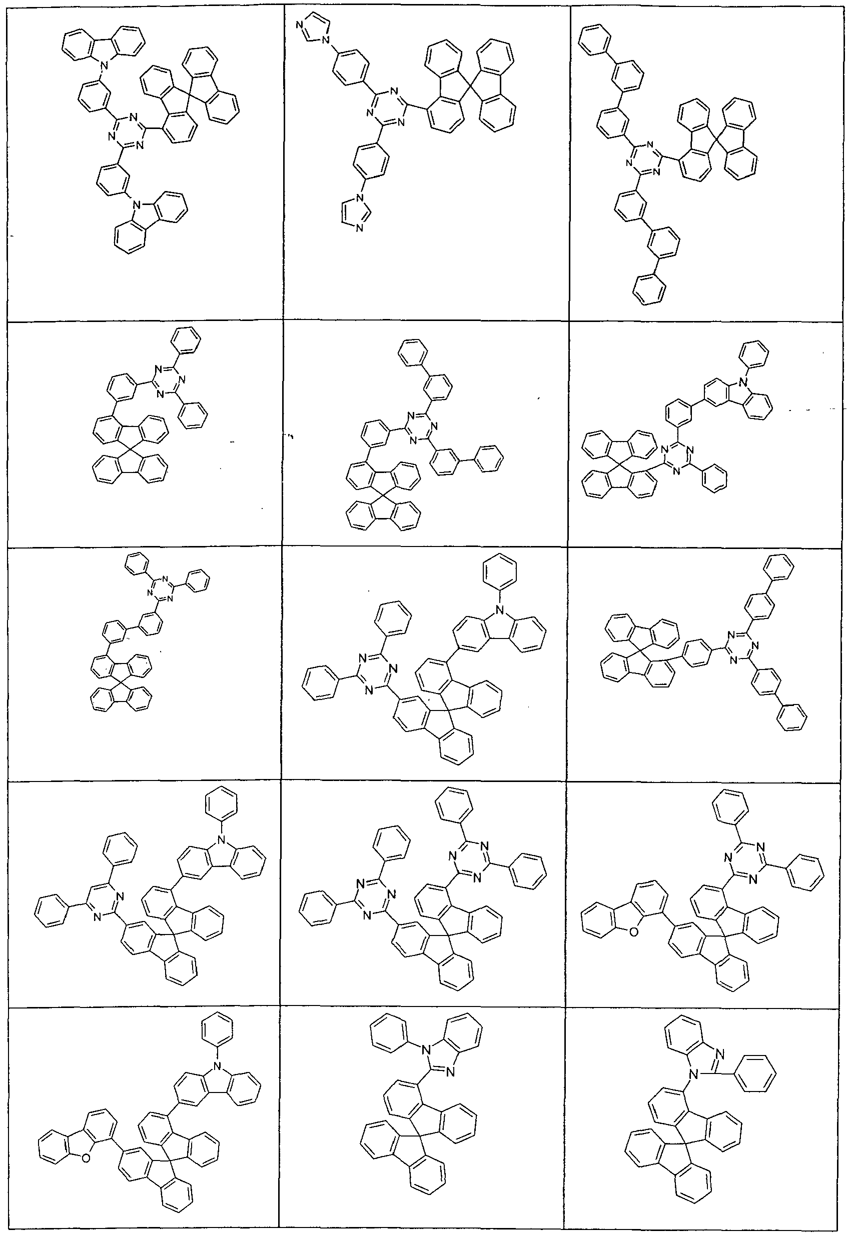

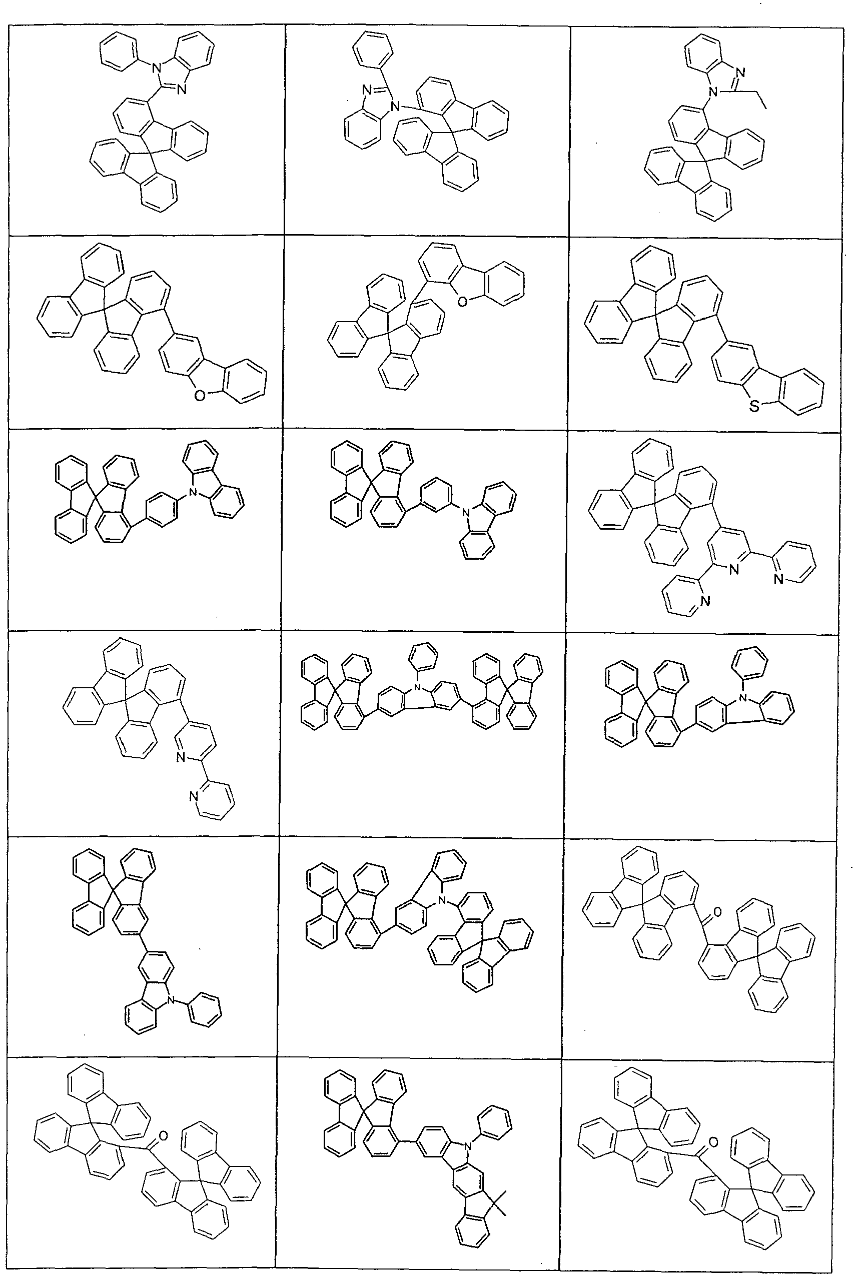

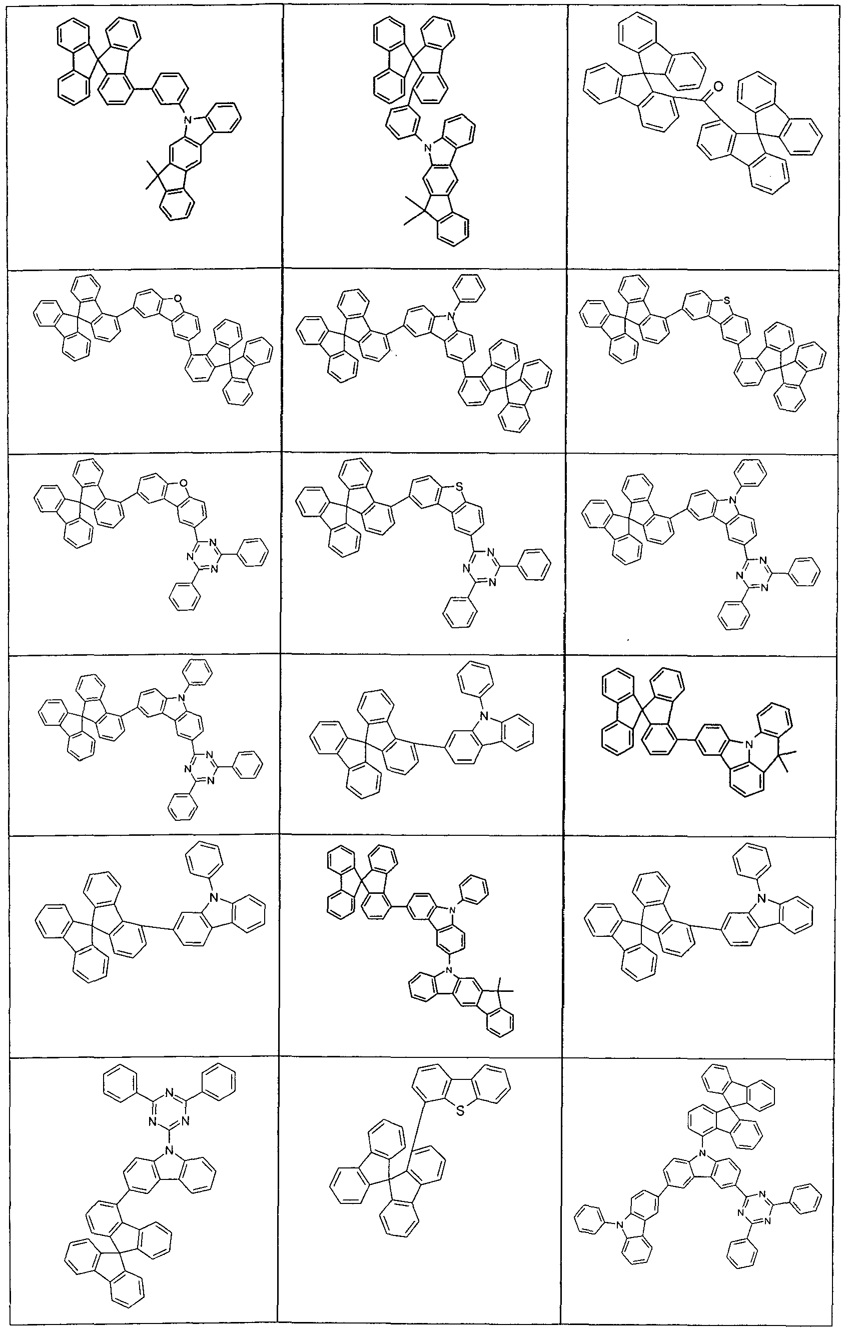

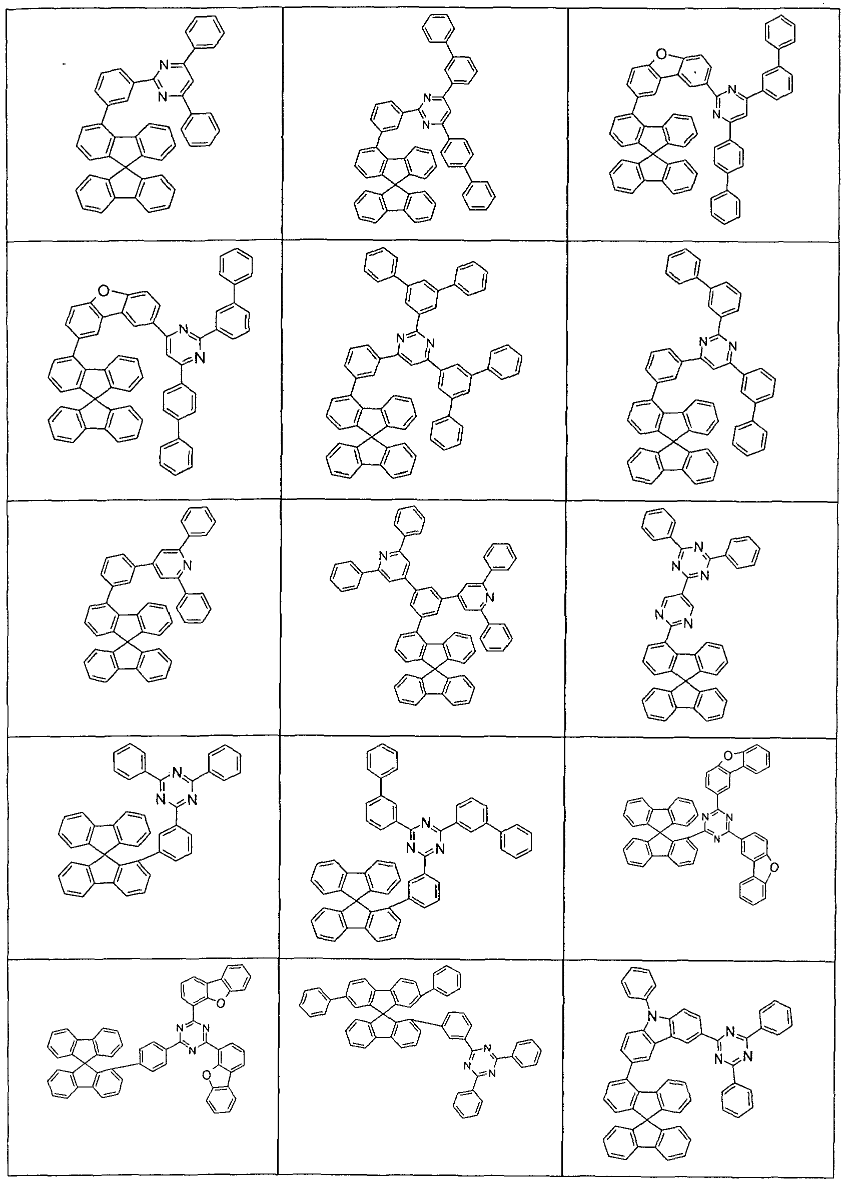

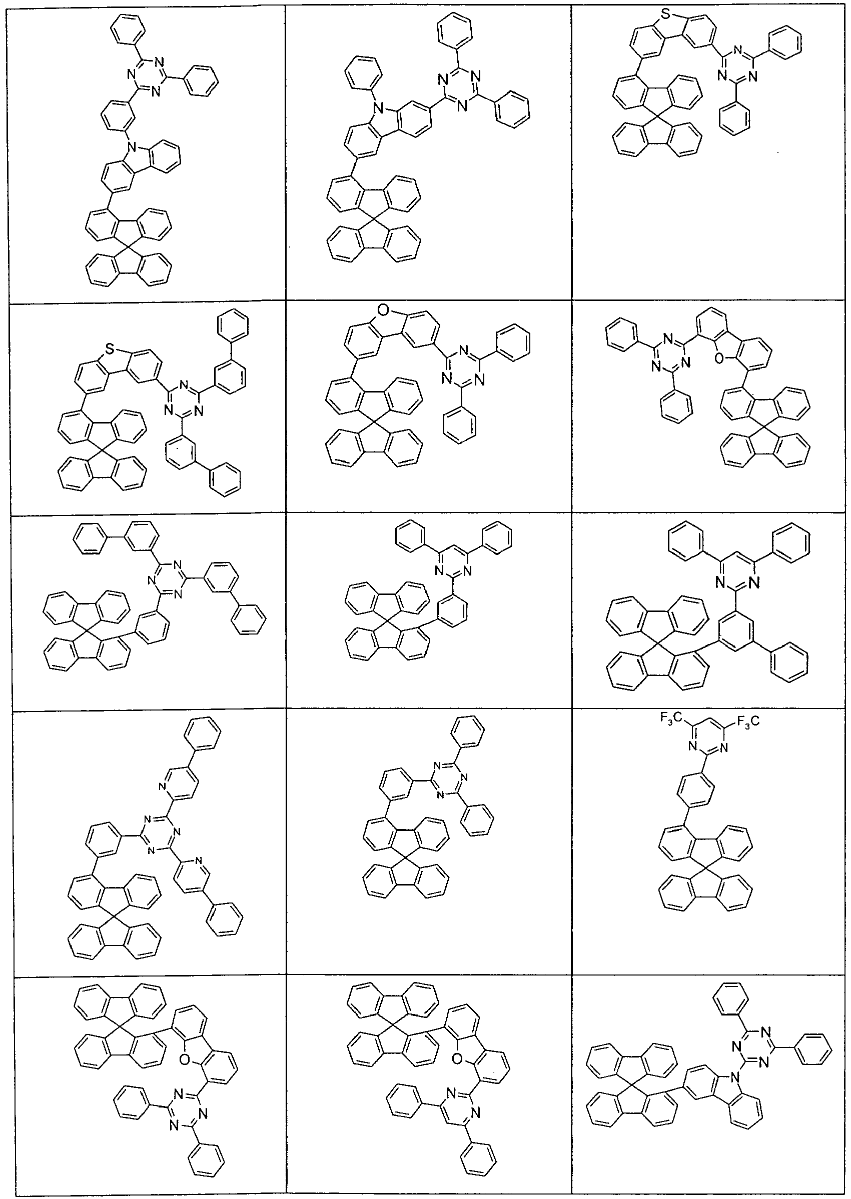

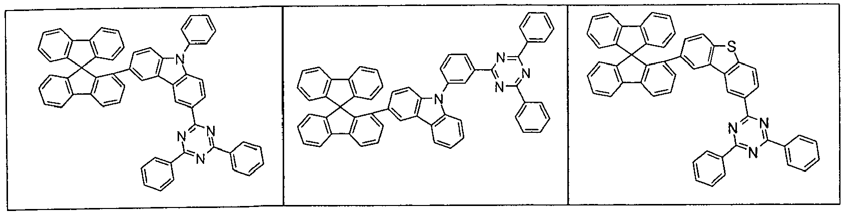

Die erfindungsgemäßen Verbindungen können nach dem Fachmann bekannten Syntheseschritten, wie z. B. Bromierung, Ullmann-Arylierung, Hartwig-Buchwald-Kupplung, etc., dargestellt werden.

Dabei gehen die Synthesen im Allgemeinen von den in 1- bzw. 4-halogenierten, insbesondere bromierten Spirobifluorenderivaten aus, gefolgt von Einführung der Gruppe -Ar bzw. -L-Ar, insbesondere durch eine metallkatalysierte Kupplungsreaktion, beispielsweise eine Suzukikupplung. Analog kann statt dem Halogen eine andere geeignete Abgangsgruppe verwendet werden, beispielsweise Tosylat oder Triflat. Die Synthese von in 1-Position mit Ar substituiertem Spirobifluoren ist in Schema 1 gezeigt. Die Synthese von in 4-Position mit Ar substituiertem Spirobifluoren ist in Schema 2 gezeigt.

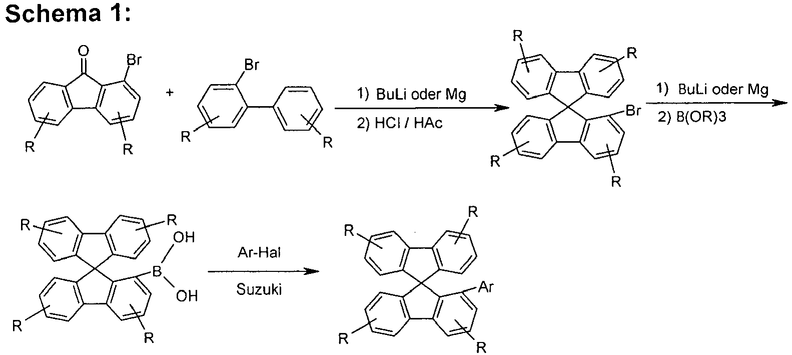

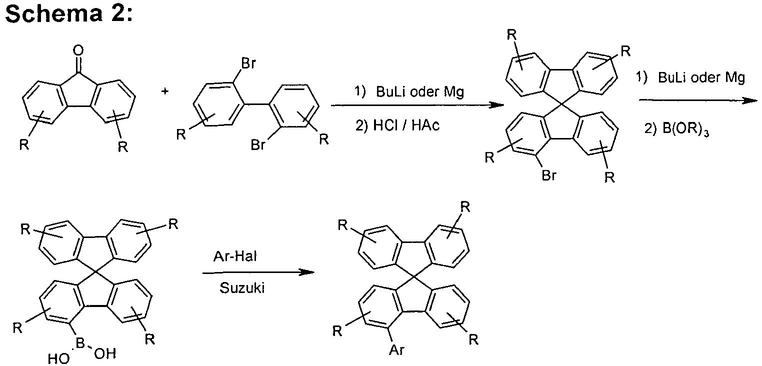

Ebenso lassen sich ganz analog auch entsprechende Verbindungen synthetisieren, in welchen die Gruppe Ar nicht direkt an das Spirobifluoren gebunden ist, sondern über eine Gruppe L, welche nicht für eine Einfachbindung steht, indem statt einem halogenierten Aromaten Ar-Hal eine entsprechende Verbindung Ar-L-Hal eingesetzt wird. Dabei steht Hal bevorzugt für Cl, Br oder I, insbesondere für Br.

Ebenso lassen sich ganz analog die halogenierten Spirobifluorenderivate einsetzen, die mit einem Boronsäurederivat der Gruppe -L-Ar gekuppelt werden.

Ein weiterer Gegenstand der vorliegenden Erfindung ist daher ein Verfahren zur Herstellung einer Verbindung gemäß Formel (1) bzw. (2), dadurch gekennzeichnet, dass die Gruppe -L-Ar durch eine metallkatalysierte Kupplungsreaktion zwischen einem 1- bzw. 4-funktionalisierten Spirobifluoren und einer funktionalisierten Gruppe -L-Ar eingeführt wird. In einer bevorzugten Ausführungsform der Erfindung handelt es sich bei dem Spirobifluorenderivat um eine halogen-funktionalisierte Verbindung und bei der Gruppe -L-Ar um eine Verbindung, welche mit einem Boronsäurederivat funktionalisiert ist. In einer weiteren bevorzugten Ausführungsform der Erfindung handelt es sich bei dem Spirobifluorenderivat um eine Verbindung, welche mit einem Boronsäurederivat funktionalisiert ist, und bei der Gruppe -L-Ar um eine halogen-funktionalisierte Verbindung.

Die erfindungsgemäßen Verbindungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine erfindungsgemäße Verbindung. Dabei gelten die oben ausgeführten Bevorzugungen ebenso für die elektronischen Vorrichtungen.

Die elektronische Vorrichtung ist bevorzugt ausgewählt aus der Gruppe bestehend aus organischen Elektrolumineszenzvorrichtungen (organischen Leuchtdioden, OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (ODSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt aber organischen Elektrolumineszenzvorrichtungen (OLEDs), besonders bevorzugt phosphoreszierenden OLEDs.

Die organischen Elektrolumineszenzvorrichtungen und die lichtemittierenden elektrochemischen Zellen können für verschiedene Anwendungen eingesetzt werden, beispielsweise für einfarbige oder mehrfarbige Displays, für Beleuchtungsanwendungen oder für medizinische und/oder kosmetische Anwendungen, beispielsweise in der Phototherapie.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Dabei ist es möglich, dass alle emittierenden Schichten fluoreszierend sind oder dass alle emittierenden Schichten phosphoreszierend sind oder dass eine oder mehrere emittierende Schichten fluoreszierend und eine oder mehrere andere Schichten phosphoreszierend sind.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. Formel (2) bzw. die bevorzugten Ausführungsformen als Elektronentransportmaterial in einer Elektronentransport- oder Lochblockierschicht oder als Matrixmaterial für fluoreszierende oder phosphoreszierende Emitter, insbesondere für phosphoreszierende Emitter. Dabei gelten die oben aufgeführten bevorzugten Ausführungsformen auch für die Verwendung der Materialien in elektronischen Vorrichtungen.

In einer bevorzugten Ausführungsform der Erfindung wird die Verbindung gemäß Formel (1) bzw. Formel (2) bzw. die bevorzugten Ausführungsformen als Elektronentransportmaterial in einer Elektronentransportschicht eingesetzt. Dabei kann die emittierende Schicht fluoreszierend oder phosphoreszierend sein. Weiterhin kann die Elektronentransportschicht direkt an die Kathode angrenzen, oder es kann eine zusätzliche Elektroneninjektionsschicht vorhanden sein, welche zwischen der Kathode und der Elektronentransportschicht vorliegt. Ebenso können mehrere Elektronentransportschichten vorliegen, von denen mindestens eine Schicht mindestens eine Verbindung gemäß Formel (1) bzw. (2) enthält.

In einer weiteren bevorzugten Ausführungsform der Erfindung wird die Verbindung gemäß Formel (1) bzw. Formel (2) bzw. die bevorzugten Ausführungsformen in einer Lochblockierschicht eingesetzt. Unter einer Lochblockierschicht wird eine Schicht verstanden, die auf Kathodenseite direkt an eine emittierende Schicht angrenzt.

In einer weiteren bevorzugten Ausführungsform der Erfindung wird die Verbindung gemäß Formel (1) bzw. Formel (2) bzw. die bevorzugten Ausführungsformen als Matrixmaterial (= Hostmaterial) für eine fluoreszierende oder phosphoreszierende Verbindung, insbesondere für eine phosphoreszierende Verbindung, in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. Formel (2) bzw. die bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand. Im Sinne dieser Anmeldung sollen alle lumineszierenden Komplexe mit Übergangsmetallen oder Lanthanoiden, insbesondere alle lumineszierenden Iridium-, Platin- und Kupferkomplexe als phosphoreszierende Verbindungen angesehen werden.

Die Mischung aus der Verbindung gemäß Formel (1) bzw. Formel (2) bzw. die bevorzugten Ausführungsformen und der emittierenden Verbindung enthält zwischen 99.9 und 1 Gew.-%, vorzugsweise zwischen 99 und 10 Gew.-%, besonders bevorzugt zwischen 97 und 60 Gew.-%, insbesondere zwischen 95 und 80 Gew.-% der Verbindung gemäß Formel (1) bzw. Formel (2) bzw. die bevorzugten Ausführungsformen bezogen auf die Gesamtmischung aus Emitter und Matrixmaterial. Entsprechend enthält die Mischung zwischen 0.1 und 99 Gew.-%, vorzugsweise zwischen 1 und 90 Gew.-%, besonders bevorzugt zwischen 3 und 40 Gew.-%, insbesondere zwischen 5 und 20 Gew.-% des Emitters bezogen auf die Gesamtmischung aus Emitter und Matrixmaterial. Die oben angegebenen Grenzen gelten insbesondere, wenn die Schicht aus Lösung aufgebracht wird. Wird die Schicht durch Vakuumverdampfung aufgebracht, gelten dieselben Zahlenwerte, wobei in diesem Fall der Prozenzanteil jeweils in Vol.-% angegeben ist.

Eine besonders bevorzugte Ausführungsform der vorliegenden Erfindung ist der Einsatz der Verbindung gemäß Formel (1) bzw. Formel (2) bzw. die bevorzugten Ausführungsformen als Matrixmaterial für einen phosphoreszierenden Emitter in Kombination mit einem weiteren Matrixmaterial. Besonders geeignete Matrixmaterialien, welche in Kombination mit den Verbindungen gemäß Formel (1) bzw. Formel (2) bzw. den bevorzugten Ausführungsformen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl), m-CBP oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 08/056746, Zinkkomplexe, z. B. gemäß EP 652273 oder WO 2009/062578, Fluorenderivate, z. B. gemäß WO 2009/124627, Diazasilol- bzw. Tetraazasilol-Derivate, z. B. gemäß WO 2010/054729, Diazaphosphol-Derivate, z. B. gemäß WO 2010/054730, oder verbrückte Carbazolderivate, z. B. gemäß US 2009/0136779, WO 2010/050778, WO 2011/042107 oder WO 2011/088877. Weiterhin ist es möglich, einen elektronisch neutralen Co-Host zu verwenden, der weder lochtransportierende noch elektronentransportierende Eigenschaften aufweist, wie beispielsweise in WO 2010/108579 beschrieben.

Ebenso ist es möglich, zwei oder mehrere phosphoreszierende Emitter in der Mischung zu verwenden. Dabei wirkt der Emitter, welcher kürzerwellig emittiert, als Co-Host in der Mischung.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Beispiele der oben beschriebenen Emitter können den Anmeldungen WO 2000/70655, WO 2001/41512, WO 2002/02714, WO 2002/15645, EP 1191613, EP 1191612, EP 1191614, WO 2005/033244, WO 2005/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/157339 oder WO 2012/007086 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Es ist weiterhin möglich, die Verbindung gemäß Formel (1) bzw. Formel (2) bzw. die bevorzugten Ausführungsformen sowohl in einer Lochblockierschicht bzw. Elektronentransportschicht als auch als Matrix in einer emittierenden Schicht zu verwenden.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. Formel (2) bzw. den bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. LITI (Light Induced Thermal Imaging, Thermotransferdruck), Ink-Jet Druck (Tintenstrahldruck), Siebdruck, Flexodruck, Offsetdruck oder Nozzle Printing, hergestellt werden. Hierfür sind lösliche Verbindungen nötig, welche beispielsweise durch geeignete Substitution erhalten werden. Diese Verfahren eignen sich insbesondere auch für die erfindungsgemäßen Verbindungen, da diese allgemein eine sehr gute Löslichkeit in organischen Lösemitteln aufweisen.

Weiterhin sind Hybridverfahren möglich, bei denen beispielsweise eine oder mehrere Schichten aus Lösung aufgebracht werden und eine oder mehrere weitere Schichten aufgedampft werden. So kann beispielsweise die emittierende Schicht aus Lösung aufgebracht werden und die Elektronentransportschicht aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Formulierung, insbesondere eine Lösung, Dispersion oder Miniemulsion, enthaltend mindestens eine Verbindung gemäß Formel (1) bzw. Formel (2) bzw. die oben ausgeführten bevorzugten Ausführungsformen und mindestens ein Lösemittel, insbesondere ein organisches Lösemittel. Wie solche Lösungen hergestellt werden können, ist dem Fachmann bekannt und beispielsweise in der WO 2002/072714, der WO 2003/019694 und der darin zitierten Literatur beschrieben.

Ein weiterer Gegenstand der vorliegenden Erfindung sind Mischungen enthaltend mindestens eine Verbindung gemäß Formel (1) bzw. Formel (2) bzw. die oben ausgeführten bevorzugten Ausführungsformen und mindestens eine weitere Verbindung. Die weitere Verbindung kann beispielsweise ein fluoreszierender oder phosphoreszierender Dotand sein, wenn die erfindungsgemäße Verbindung als Matrixmaterial verwendet wird. Die Mischung kann dann auch zusätzlich noch ein weiteres Material als zusätzliches Matrixmaterial enthalten.

Die erfindungsgemäßen Verbindungen und die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochblockier- bzw. Elektronentransportschicht in einer organischen Elektrolumineszenzvorrichtung. Dabei eignen sie sich insbesondere auch für die Verwendung in einer Lochblockierschicht, die direkt an eine phosphoreszierende emittierende Schicht angrenzt, da die erfindungsgemäßen Verbindungen nicht die Lumineszenz löschen.
2. Die erfindungsgemäßen Verbindungen, eingesetzt als Matrixmaterial für fluoreszierende oder phosphoreszierende Emitter führen zu sehr hohen Effizienzen sowie zu langen Lebensdauern. Dies gilt insbesondere, wenn die Verbindungen als Matrixmaterial zusammen mit einem weiteren Matrixmaterial und einem phosphoreszierenden Emitter eingesetzt werden.
3. Die erfindungsgemäßen Verbindungen, eingesetzt in organischen Elektrolumineszenzvorrichtungen, führen zu hohen Effizienzen und zu steilen Strom-Spannungs-Kurven mit niedrigen Einsatz- und Betriebsspannungen.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Edukte können von ALDRICH (Kaliumfluorid (sprühgetrocknet), Tri-*tert*-butylphosphin, Palladium(II)acetat) bezogen werden. 3-Chlor-5,6-diphenyl-1,2,4-triazin wird analog zu EP 577559 dargestellt. 2',7'-Di-*tert*-butyl-spiro-9,9'-bifluoren-2,7-bisboronsäureglycolester wird nach WO 2002/077060 und 2-Chlor-4,6-diphenyl-1,3,5-triazin nach US 5,438138 dargestellt. Spiro-9,9'-bifluoren-2,7-bis(boronsäureglycolester) wird analog zu WO 2002/077060 dargestellt. Die Nummern bei den literaturbekannten Edukten, die teilweise in eckigen Klammern angegeben sind, sind die entsprechenden CAS-Nummern.

### Beispiel 1: 1-Brom-spiro-9,9'-bifluoren

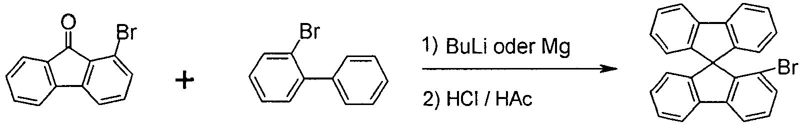

Aus mit Jod aktivierten 2.7 g (110 mmol) Magnesiumspänen und einer Mischung aus 25.6 g (110 mmol) 2-Brombiphenyl, 0.8 ml 1,2-Dichlorethan, 50 ml 1,2-Dimethoxyethan, 400 ml THF und 200 ml Toluol wird unter Begleitheizen mit einem 70 °C warmen Ölbad das entsprechende Grignard-Reagenz dargestellt. Nachdem das Magnesium vollständig abreagiert hat, lässt man auf Raumtemperatur erkalten und tropft dann eine Lösung von 25.9 g (100 mmol) 1-Brom-fluorenon, [36804-63-4] in 500 ml THF zu, erwärmt die Reaktionsmischung für 4 h auf 50 °C und rührt dann 12 h bei Raumtemperatur nach. Man gibt 100 ml Wasser zu, rührt kurz nach, trennt die organische Phase ab und entfernt das Lösungsmittel im Vakuum. Der Rückstand wird in der Wärme bei 40 °C in 500 ml Eisessig suspendiert, die Suspension wird mit 0.5 ml konz. Schwefelsäure versetzt, und anschließend 2 h bei 100 °C nachgerührt. Nach Erkalten saugt man vom ausgefallenen Feststoff ab, wäscht diesen einmal mit 100 ml Eisessig, dreimal mit je 100 ml Ethanol und kristallisiert abschließend aus Dioxan um. Ausbeute: 26.9 g (68 mmol), 68 %; Reinheit ca. 98 % nch ¹H-NMR.

**Analog wird die folgende Verbindung erhalten:**

| | Edukt 1 | Edukt 2 | Produkt | Ausbeute |
|---|---|---|---|---|
| 1a | 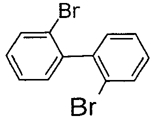 | 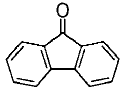 | 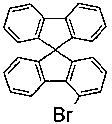 | 90% |
| | 13029-09-9 | 486-25-9 | 1161009-88-6 | |

### Beispiel 2: Synthese von 4-(4,6-diphenyl-1,3,5-triazin-2-yl)spiro-9,9'-bifluoren

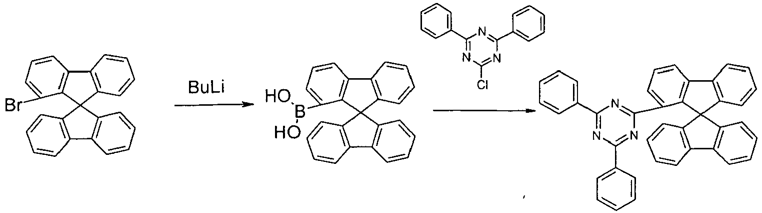

### Schritt 1) Synthese von Spiro-9,9'-bifluoren-1-boronsäure

Eine auf-78 °C gekühlte Lösung von 106 g (270 mmol) 1-Brom-9-spirobifluoren in 1500 ml Diethylether wird tropfenweise mit 110 ml (276 mmol) n-Buthyllithium (2.5 M in Hexan) versetzt. Die Reaktionsmischung wird 30 min. bei -78 °C gerührt. Man lässt auf Raumtemperatur kommen, kühlt erneut auf -78 °C und versetzt dann schnell mit einer Mischung von 40 ml (351 mmol) Trimethylborat in 50 ml Diethylether. Nach Erwärmen auf -10 °C wird mit 135 ml 2 N Salzsäure hydrolysiert. Die organische Phase wird abgetrennt, mit Wasser gewaschen, über Natriumsulfat getrocknet und zur Trockene eingeengt. Der Rückstand wird in 300 ml n-Heptan aufgenommen, der farblose Feststoff wird abgesaugt, mit n-Heptan gewaschen und im Vakuum getrocknet. Ausbeute: 94,5 g (255 mmol), 99 % d. Th.; Reinheit: 99 % nach HPLC.

**Analog wird die folgende Verbindung erhalten:**

| | Edukt 1 | Produkt | Ausbeute |
|---|---|---|---|
| 2a | 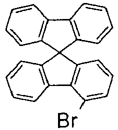 | 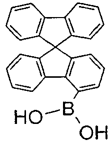 | 83% |

### Schritt 2) Synthese von 1-(4,6-Diphenyl-1,3,5-triazin-2-yl)-spiro-9,9'-bifluoren

56,8 g (110 mmol) Spiro-9,9'-bifluoren-1-boronsäure, 29.5 g (110.0 mmol) 2-Chlor-4,6-diphenyl-1,3,5-triazin und 44.6 g (210.0 mmol) Trikaliumphosphat werden in 500 mL Toulol, 500 mL Dioxan und 500 mL Wasser suspendiert. Zu dieser Suspension werden 913 mg (3.0 mmol) Tri-o-tolylphosphin und dann 112 mg (0.5 mmol) Palladium(II)acetat gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol und aus Dichlormethan / *iso*-Propanol umkristallisiert und abschließend im Hochvakuum (p = 5 x 10⁻⁵ mbar, T = 377 °C) sublimiert. Die Ausbeute beträgt 38.7 g (43,5 mmol), entsprechend 87 % der Theorie.

**Analog werden die folgenden Verbindungen erhalten:**

| | Edukt 1 | Edukt 2 | Produkt | Ausbeute |
|---|---|---|---|---|
| 2b | 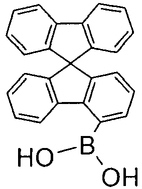 | 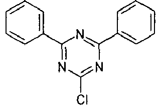 | 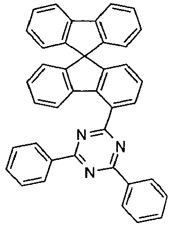 | 81% |
| | | 3842-55-5 | | |
| 2c | 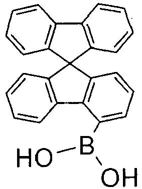 | 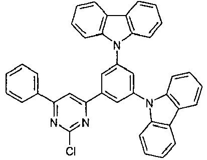 | 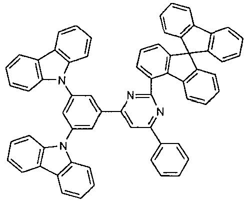 | 80 % |
| | | 1333505-18-2 | | |
| 2d | 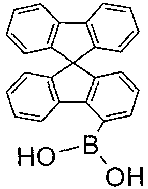 | 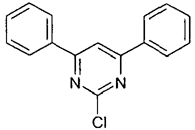 | 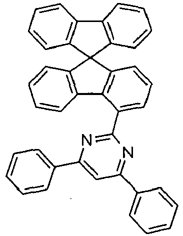 | 79% |
| | | 2915-16-4 | | |
| 2e | 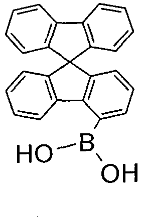 | 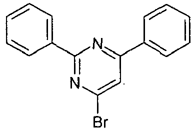 | 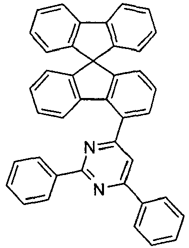 | 77% |
| | | 40734-4-5 | | |
| 2f | 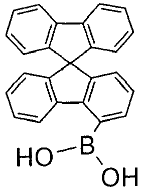 | 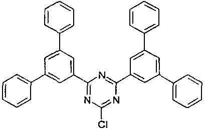 | 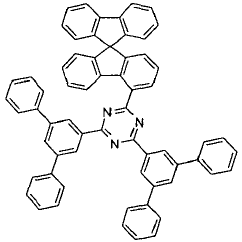 | 87% |
| | | 1205748-51-1 | | |
| 2g | 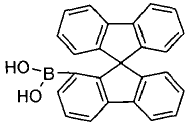 | 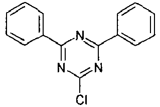 | 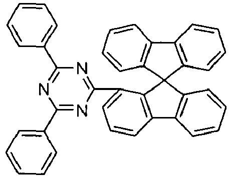 | 76% |
| | | 3842-55-5 | | |
| 2h | 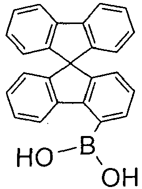 | 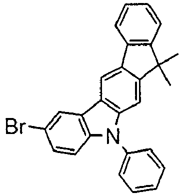 | 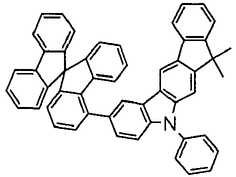 | 65% |
| | | 1257220-44-2 | | |
| 2i | 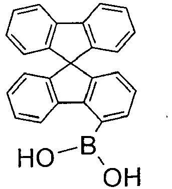 | 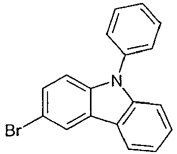 | 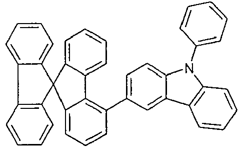 | 79% |
| | | 1153-85-1 | | |
| 2j | 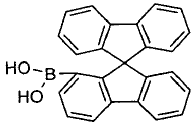 | 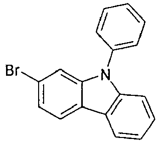 | 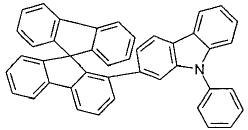 | 69% |
| | | 94994-62-4 | | |
| 2k | 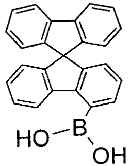 | 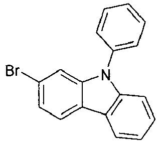 | 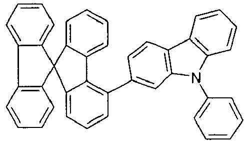 | 73% |
| | | 94994-62-4 | | |

### Beispiel 3: Herstellung der OLEDs

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1 bis E3 (siehe Tabellen 1 und 2) werden die Daten verschiedener OLEDs vorgestellt. Glasplättchen, die mit strukturiertem ITO (Indium Zinn Oxid) der Dicke 50 nm beschichtet sind werden zur verbesserten Prozessierung mit 20 nm PEDOT:PSS beschichtet (Poly(3,4-ethylendioxythiophen)poly(styrolsulfonat), bezogen als CLEVIOS™ P VP Al 4083 von Heraeus Precious Metals GmbH Deutschland, aus wässriger Lösung aufgeschleudert). Diese beschichteten Glasplättchen bilden die Substrate, auf welchen die OLEDs aufgebracht werden. Die OLEDs haben prinzipiell folgenden Schichtaufbau: Glassubstrat / ITO/ optionale Lochinjektionsschicht (HIL) / Lochtransportschicht (HTL) / optionale Zwischenschicht (IL) / Elektronenblockerschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die weiteren zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus mindestens einem Matrixmaterial (auch Hostmaterial oder Wirtsmaterial) und einem emittierenden Dotierstoff (Dotand oder Emitter), der dem Matrixmaterial bzw. den Matrixmaterialien durch Coverdampfung in einem bestimmten Volumenanteil beigemischt wird. Eine Angabe wie Host1:Host3:TEG1 (30%:60%:10%) bedeutet hierbei, dass das Material Host3 in einem Volumenanteil von 60%, Host1 in einem Anteil von 30% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 und LE1000 bezeichnen die Strom- bzw. Leistungseffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiele V1 bis V4 sind Vergleichsbeispiele gemäß dem Stand der Technik, die Beispiele E1 bis E3 zeigen Daten von OLEDs mit erfindungsgemäßen Materialien.

Im Folgenden werden einige der Beispiele näher erläutert, um die Vorteile der erfindungsgemäßen Verbindungen zu verdeutlichen. Es sei jedoch darauf hingewiesen, dass dies nur eine Auswahl der in Tabelle 2 gezeigten Daten darstellt.

### Verwendung von erfindungsgemäßen Verbindungen in der Elektronentransportschicht

Setzt man die Verbindung Host2 als Elektronentransportschicht ein, so erhält man eine niedrige Betriebsspannung von 3.5 V und eine sehr gute Quanteneffizienz von fast 17 % (Beispiel E2), bei Einsatz der Verbindung Host1 gemäß dem Stand der Technik sind diese Werte schlechter (Beispiel V2).

### Verwendung von erfindungsgemäßen Verbindungen als Matrixmaterialien in phosphoreszierenden OLEDs

Bei Verwendung von erfindungsgemäßen Materialien als Einzelmatrixmaterial in Kombination mit dem grünen Dotanden TEG1 erhält man sehr niedrige Betriebsspannungen von 4.4 V und sehr gute Quanteneffizienzen von 15.6 % (Beispiel E1), während die Spannung im Vergleichsbeispiel V1 bei fast gleicher Effizienz höher ist.

Auch in einem Mixed-Matrix System erhält man mit erfindungsgemäßen Materialien sehr gute Leistungsdaten. In Kombination mit dem Material Host3 z. B. erhält man eine sehr niedrige Betriebsspannung von 3.6 V (Beispiel E3), während diese im Vergleichsbeispiel V4 deutlich höher ist.

**Tabelle 1: Aufbau der OLEDs**

| Bsp. | HIL Dicke | HTL Dicke | IL Dicke | EBL Dicke | EML Dicke | HBL Dicke | ETL Dicke | EIL Dicke |
|---|---|---|---|---|---|---|---|---|
| V1 | --- | HIM1 70nm | HATCN 5nm | HTM1 90nm | Host1:TEG1 (90%:10%) 30nm | HBM1 10nm | ETM1:LiQ (50%:50%) 30nm | --- |
| V2 | --- | HIM1 70nm | HATCN 5nm | HTM2 90nm | HBM1:TEG1 (90%:10%) 30nm | | Host1:LiQ (50%:50%) 30nm | --- |
| V3 | --- | HIM1 70nm | HATCN 5nm | HTM1 90nm | Host4:Host3:TEG1 (30%:60%:10%) 30nm | HBM1 10nm | ETM1:LiQ (50%:50%) 30nm | --- |
| V4 | --- | HIM1 70nm | HATCN 5nm | HTM1 90nm | Host1:Host3:TEG1 (30%:60%:10%) 30nm | HBM1 10nm | ETM1:LiQ (50%:50%) 30nm | --- |
| E1 | --- | HIM1 70nm | HATCN 5nm | HTM1 90nm | Host2:TEG1 (90%:10%) 30nm | HBM1 10nm | ETM1:LiQ (50%:50%) 30nm | --- |
| E2 | --- | HIM1 70nm | HATCN 5nm | HTM2 90nm | HBM1:TEG1 (90%:10%) 30nm | | Host2:LiQ (50%:50%) 30nm | --- |
| E3 | --- | HIM1 70nm | HATCN 5nm | HTM1 90nm | Host2:Host3:TEG1 (30%:60%:10%) 30nm | HBM1 10nm | ETM1:LiQ (50%:50%) 30nm | --- |

**Tabelle 2: Daten der OLEDs**

| Bsp. | U1000 (V) | SE1000 (cd/A) | LE1000 (lm/W) | EQE1000 (%) | CIE x/y bei 1000 cd/M1 |
|---|---|---|---|---|---|
| V1 | 4.8 | 55 | 36 | 15.9 | 0.39/0.58 |
| V2 | 3.8 | 56 | 46 | 16.0 | 0.38/0.59 |
| V3 | 4.0 | 52 | 41 | 14.7 | 0.39/0.58 |
| V4 | 3.9 | 53 | 43 | 15.0 | 0.39/0.58 |
| E1 | 4.4 | 54 | 39 | 15.6 | 0.39/0.58 |
| E2 | 3.5 | 59 | 53 | 16.9 | 0.38/0.59 |
| E3 | 3.6 | 54 | 46.5 | 15.0 | 0.38/0.59 |

**Tabelle 3: Strukturformeln der Materialien für die OLEDs**

| | |
|---|---|
| 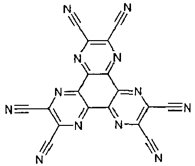 | 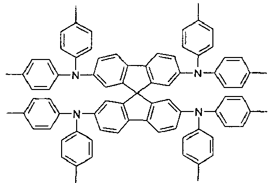 |
| HATCN | HIM1 |
| 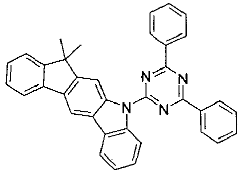 | 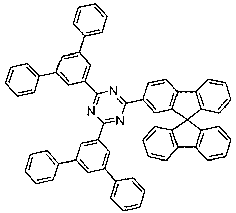 |
| HBM1 | ETM1 (Stand der Technik) |
| 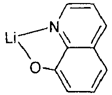 | 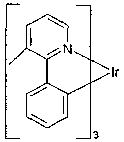 |
| LiQ | TEG1 |
| 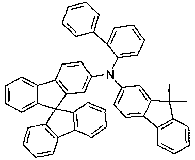 | 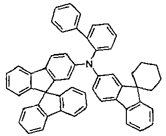 |
| HTM1 | HTM2 |
| 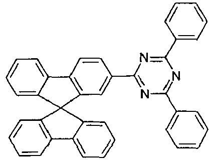 | 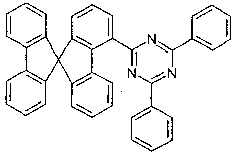 |
| Host 1 (Stand der Technik) | Host 2 (erfindungsgemäß) |
| 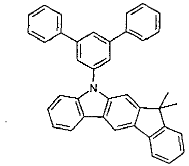 | 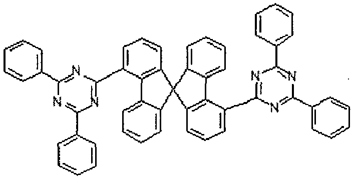 |
| Host 3 | Host 4 (Stand der Technik) |

## Patentansprüche

1. Verbindung gemäß Formel (1) oder (2), 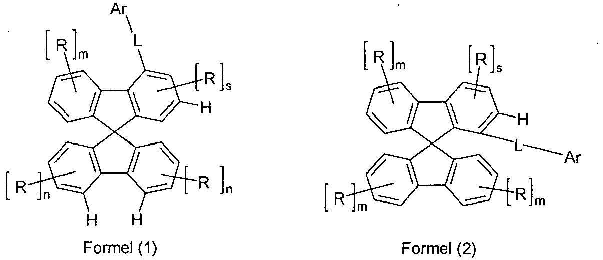 wobei für die verwendeten Symbole und Indizes gilt:
Ar ist ein heteroaromatisches Ringsystem mit 5 bis 40 aromatischen Ringatomen, welches über ein Kohlenstoffatom an L gebunden ist, wenn L für eine Einfachbindung steht, und welches über ein Kohlenstoffatom oder ein Stickstoffatom an L gebunden ist, wenn L ungleich einer Einfachbindung ist, und welches durch einen oder mehrere Reste R¹ substituiert sein kann; oder Ar ist ein aromatisches Ringsystem mit 6 bis 40 aromatischen Ringatomen, welches durch einen oder mehrere Reste R¹ substituiert sein kann, wenn L für C(=O) steht;
L ist eine Einfachbindung, C(=O) oder ein aromatisches Ringsystem mit 5 bis 24 aromatischen Ringatomen, welches durch einen oder mehrere Reste R substituiert sein kann;
R, R¹ ist bei jedem Auftreten gleich oder verschieden ausgewählt aus der Gruppe bestehend aus H, D, F, Cl, Br, I, CN, Si(R²)₃, einer geradkettigen Alkyl-, Alkoxy- oder Thioalkylgruppe mit 1 bis 40 C-Atomen oder einer verzweigten oder cyclischen Alkyl-, Alkoxy- oder Thioalkylgruppe mit 3 bis 40 C-Atomen, die jeweils mit einem oder mehreren Resten R² substituiert sein kann, wobei jeweils eine oder mehrere nicht-benachbarte CH₂-Gruppen durch Si(R²)₂, C=NR², P(=O)(R²), SO, SO₂, NR², O, S oder CONR² ersetzt sein können und wobei ein oder mehrere H-Atome durch D, F, Cl, Br oder I ersetzt sein können, einem aromatischen oder heteroaroma-tischen Ringsystem mit 6 bis 40 C-Atomen, das jeweils mit einem oder mehreren Resten R² substituiert sein kann, einer Aryloxygruppe mit 5 bis 60 aromatischen Ringatomen, die mit einem oder mehreren Resten R² substituiert sein kann, oder einer Aralkylgruppe mit 5 bis 60 aromatischen Ringatomen, die jeweils mit einem oder mehreren Resten R² substituiert sein kann, wobei optional zwei oder mehr benachbarte Substituenten R bzw. R¹ ein mono- oder polycyclisches, aliphatisches Ringsystem bilden können, das mit einem oder mehreren Resten R² substituiert sein kann;
R² ist ausgewählt aus der Gruppe bestehend aus H, D, F, einem aliphatischem Kohlenwasserstoffrest mit 1 bis 20 C-Atomen oder einem aromatischem oder heteroaromatischen Ringsystem mit 5 bis 30 C-Atomen, in dem ein oder mehrere H-Atome durch D oder F ersetzt sein können, wobei zwei oder mehr benachbarte Substituenten R² miteinander ein mono- oder polycyclisches, aliphatisches, Ringsystem bilden können;
s ist 0, 1 oder 2;
m ist bei jedem Auftreten gleich oder verschieden 0, 1, 2, 3 oder 4;
n ist bei jedem Auftreten gleich oder verschieden 0, 1, 2 oder 3.

2. Verbindung nach Anspruch 1, ausgewählt aus den Verbindungen der Formel (1a) und Formel (2a), 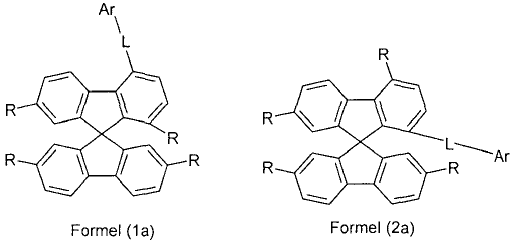 wobei die verwendeten Symbole die in Anspruch 1 genannten Bedeutungen aufweisen.

3. Verbindung nach Anspruch 1 oder 2, ausgewählt aus den Verbindungen der Formeln (1b) und (2b), 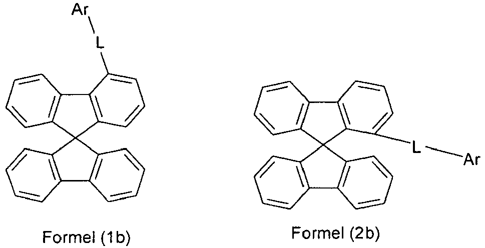 wobei die verwendeten Symbole die in Anspruch 1 genannten Bedeutungen aufweisen.

4. Verbindung nach einem oder mehreren der Ansprüche 1 bis 3, **dadurch gekennzeichnet, dass** L ausgewählt ist aus einer Einfachbindung, C(=O) oder einem aromatischen Ringsystem mit 6 bis 12 aromatischen Ringatomen, welches mit einem oder mehreren Resten R substituiert sein kann.

5. Verbindung nach einem oder mehreren der Ansprüche 1 bis 4, **dadurch gekennzeichnet, dass** L ausgewählt ist aus der Gruppe bestehend aus einer Einfachbindung oder einer ortho-, meta- oder para-verknüpfte Phenylengruppe, welche mit einem oder mehreren Resten R substituiert sein kann, bevorzugt aber unsubstituiert ist.

6. Verbindung nach einem oder mehreren der Ansprüche 1 bis 5, **dadurch gekennzeichnet, dass** Ar ein heteroaromatisches Ringsystem mit 5 bis 24 aromatischen Ringatomen darstellt, insbesondere mit 5 bis 13 aromatischen Ringatomen, das jeweils durch einen oder mehrere Reste R¹ substituiert sein kann; oder dass Ar ein aromatisches Ringsystem mit 6 bis 24 aromatischen Ringatomen darstellt, das durch einen oder mehrere Reste R¹ substituiert sein kann, wenn L für C(=O) steht.

7. Verbindung nach einem oder mehreren der Ansprüche 1 bis 6, **dadurch gekennzeichnet, dass** Ar ausgewählt ist aus der Gruppe bestehend aus Triazin, Pyrimidin, Pyrazin, Pyridazin, Pyridin, Pyrazol, Imidazol, Oxazol, Oxadiazol, Thiazol, Benzimidazol, Benzofuran, Benzothiophen, Indol, Dibenzofuran, Dibenzothiophen, Carbazol, Indenocarbazol und Indolocarbazol, wobei diese Gruppen jeweils durch einen oder mehrere Reste R¹ substituiert sein können; oder dass Ar, wenn L für C(=O) steht, ausgewählt ist aus der Gruppe bestehend aus Phenyl, Biphenyl, ortho-, meta- oder para-Terphenyl, ortho-, meta-, para- oder verzweigtes Quaterphenyl, 1-, 2-, 3- oder 4-Fluorenyl oder 1-, 2-, 3- oder 4-Spirobifluorenyl, welches jeweils mit einem oder mehreren Resten R¹ substituiert sein kann.

8. Verbindung nach einem oder mehreren der Ansprüche 1 bis 7, **dadurch gekennzeichnet, dass** Ar ausgewählt aus den Strukturen der Formeln (Ar-1) bis (Ar-24), 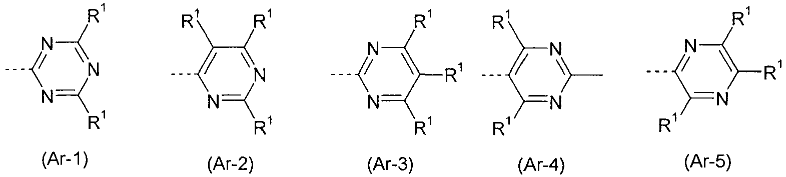 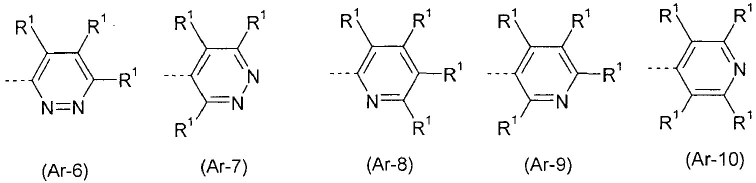 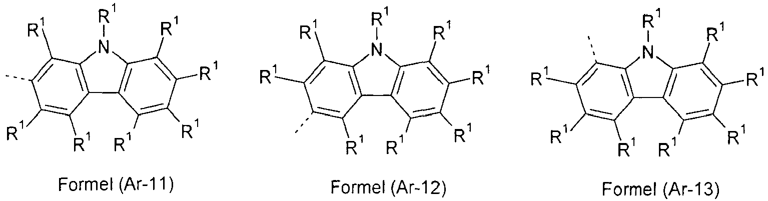 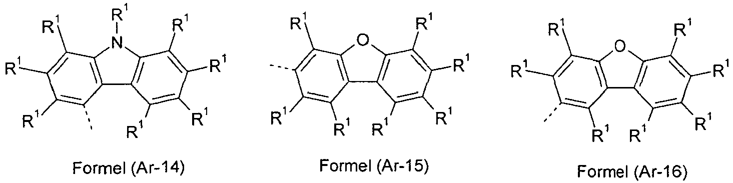 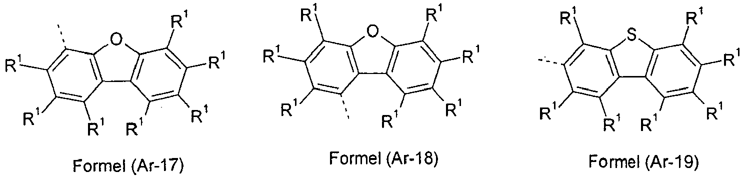 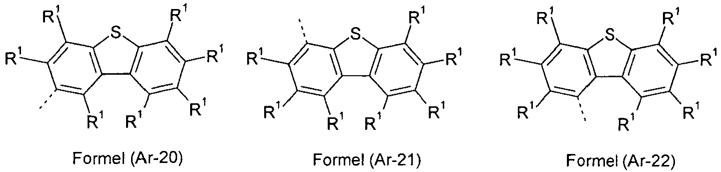 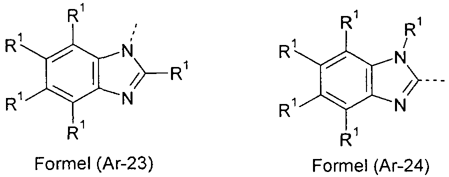 wobei die gestrichelte Bindung die Bindung an L andeutet und R¹ die in Anspruch 1 genannten Bedeutungen aufweist.

9. Verbindung nach einem oder mehreren der Ansprüche 1 bis 8, **dadurch gekennzeichnet, dass** R bzw. R¹ gleich oder verschieden bei jedem Auftreten ausgewählt sind aus der Gruppe bestehend aus H, D, F, CN, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch F ersetzt sein können, oder einem aromatischen oder heteroaromatischen Ringsystem mit 6 bis 24 aromatischen Ringatomen, das jeweils mit einem oder mehreren Resten R² substituiert sein kann.

10. Verbindung nach einem oder mehreren der Ansprüche 1 bis 9, **dadurch gekennzeichnet, dass** für die verwendeten Symbole gilt:
L ist eine Einfachbindung, C(=O) oder ein aromatisches Ringsystem mit 6 bis 12 aromatischen Ringatomen, welches mit einem oder mehreren Resten R substituiert sein kann;
Ar ist ein heteroaromatisches Ringsystem mit 5 bis 13 aromatischen Ringatomen, das durch einen oder mehrere Reste R¹ substituiert sein kann, wobei Ar über ein Kohlenstoffatom an L gebunden ist, wenn L für eine Einfachbindung stehtl, oder über ein Kohlenstoff- oder Stickstoffatom an L gebunden ist, wenn L ungleich einer Einfachbindung ist; oder ist ein aromatisches Ringsystem mit 6 bis 24 aromatischen Ringatomen, das durch einen oder mehrere Reste R¹ substituiert sein kann, wenn L für C(=O) steht;
R, R¹ ist gleich oder verschieden bei jedem Auftreten ausgewählt aus der Gruppe bestehend aus H, D, F, CN, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, die jeweils mit einem oder mehreren Resten R² substituiert sein kann, wobei eine oder mehrere nicht-benachbarte CH₂-Gruppen durch O ersetzt sein können und wobei ein oder mehrere H-Atome durch F ersetzt sein können, einem aromatischen oder heteroaromatischen Ringsystem mit 6 bis 24 aromatischen Ringatomen, das jeweils mit einem oder mehreren Resten R² substituiert sein kann.

11. Verfahren zur Herstellung einer Verbindung nach einem oder mehreren der Ansprüche 1 bis 10, **dadurch gekennzeichnet, dass** die Gruppe -L-Ar durch eine metallkatalysierte Kupplungsreaktion zwischen einem 1- bzw. 4-funktionalisierten Spirobifluoren und einer funktionalisierten Gruppe -L-Ar eingeführt wird.

12. Formulierung, enthaltend mindestens eine Verbindung nach einem oder mehreren der Ansprüche 1 bis 10, insbesondere eine Lösung, Dispersion oder Miniemulsion, enthaltend mindestens eine Verbindung nach einem oder mehreren der Ansprüche 1 bis 10 und mindestens ein Lösemittel, oder eine Mischung, enthaltend mindestens eine Verbindung nach einem oder mehreren der Ansprüche 1 bis 10 und mindestens eine fluoreszierende oder phosphoreszierende Verbindung.

13. Verwendung einer Verbindung nach einem oder mehreren der Ansprüche 1 bis 10 oder einer Formulierung nach Anspruch 12 in einer elektronischen Vorrichtung, insbesondere in einer organischen Elektrolumineszenzvorrichtung.

14. Elektronische Vorrichtung enthaltend mindestens eine Verbindung nach einem oder mehreren der Ansprüche 1 bis 10 oder mindestens eine Formulierung nach Anspruch 12, wobei die elektronische Vorrichtung bevorzugt ausgewählt ist aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, farbstoffsensibilisierten organischen Solarzellen, organischen optischen Detektoren, organischen Photorezeptoren, organischen Feld-Quench-Devices, lichtemittierenden elektrochemischen Zellen, organischen Laserdioden und Organic Plasmon Emitting Devices.

15. Elektronische Vorrichtung nach Anspruch 14, wobei es sich um eine organische Elektrolumineszenzvorrichtung handelt, **dadurch gekennzeichnet, dass** die Verbindung nach einem oder mehreren der Ansprüche 1 bis 10 als Elektronentransportmaterial in einer Elektronentransport- oder Lochblockierschicht oder als Matrixmaterial für fluoreszierende oder phosphoreszierende Emitter in einer emittierenden Schicht eingesetzt wird.

## Claims

1. Compound of the formula (1) or (2), 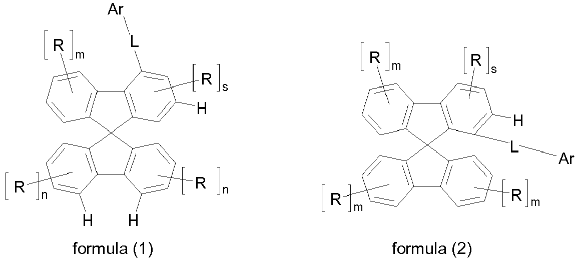 where the following applies to the symbols and indices used:
Ar is a heteroaromatic ring system having 5 to 40 aromatic ring atoms which is bonded to L via a carbon atom if L stands for a single bond, and which is bonded to L via a carbon atom or a nitrogen atom if L is not equal to a single bond, and which may be substituted by one or more radicals R¹; or Ar is an aromatic ring system having 6 to 40 aromatic ring atoms, which may be substituted by one or more radicals R¹, if L stands for C(=O);
L is a single bond, C(=O) or an aromatic ring system having 5 to 24 aromatic ring atoms, which may be substituted by one or more radicals R;
R, R¹ are selected on each occurrence, identically or differently, from the group consisting of H, D, F, Cl, Br, I, CN, Si(R²)₃, a straight-chain alkyl, alkoxy or thioalkyl group having 1 to 40 C atoms or a branched or cyclic alkyl, alkoxy or thioalkyl group having 3 to 40 C atoms, which may in each case be substituted by one or more radicals R², where in each case one or more non-adjacent CH₂ groups may be replaced by Si(R²)₂, C=NR², P(=O)(R²), SO, SO₂, NR², O, S or CONR² and where one or more H atoms may be replaced by D, F, Cl, Br or I, an aromatic or heteroaromatic ring system having 6 to 40 C atoms, which may in each case be substituted by one or more radicals R², an aryloxy group having 5 to 60 aromatic ring atoms, which may be substituted by one or more radicals R², or an aralkyl group having 5 to 60 aromatic ring atoms, which may in each case be substituted by one or more radicals R², where two or more adjacent substituents R or R¹ may optionally form a mono- or polycyclic, aliphatic ring system, which may be substituted by one or more radicals R²;
R² is selected from the group consisting of H, D, F, an aliphatic hydrocarbon radical having 1 to 20 C atoms or an aromatic or heteroaromatic ring system having 5 to 30 C atoms, in which one or more H atoms may be replaced by D or F, where two or more adjacent substituents R² may form a mono- or polycyclic, aliphatic ring system with one another;
s is 0, 1 or 2;
m is on each occurrence, identically or differently, 0, 1, 2, 3 or 4;
n is on each occurrence, identically or differently, 0, 1, 2 or 3.

2. Compound according to Claim 1, selected from the compounds of the formula (1a) and formula (2a), 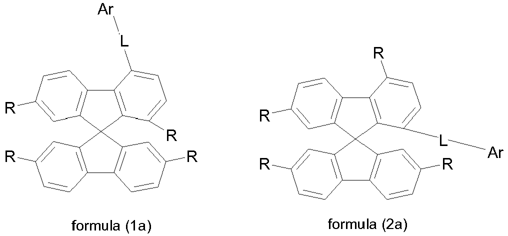 where the symbols used have the meanings given in Claim 1.

3. Compound according to Claim 1 or 2, selected from the compounds of the formulae (1b) and (2b), 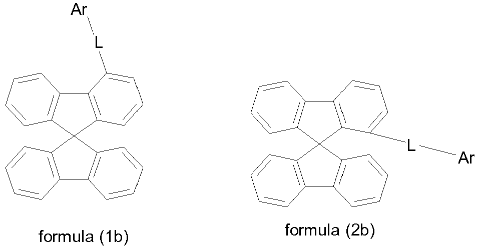 where the symbols used have the meanings given in Claim 1.

4. Compound according to one or more of Claims 1 to 3, **characterised in that** L is selected from a single bond, C(=O) or an aromatic ring system having 6 to 12 aromatic ring atoms, which may be substituted by one or more radicals R.

5. Compound according to one or more of Claims 1 to 4, **characterised in that** L is selected from the group consisting of a single bond or an ortho-, meta- or para-linked phenylene group, which may be substituted by one or more radicals R, but is preferably unsubstituted.

6. Compound according to one or more of Claims 1 to 5, **characterised in that** Ar represents a heteroaromatic ring system having 5 to 24 aromatic ring atoms, in particular having 5 to 13 aromatic ring atoms, which may in each case be substituted by one or more radicals R¹; or **in that** Ar represents an aromatic ring system having 6 to 24 aromatic ring atoms, which may be substituted by one or more radicals R¹, if L stands for C(=O).

7. Compound according to one or more of Claims 1 to 6, **characterised in that** Ar is selected from the group consisting of triazine, pyrimidine, pyrazine, pyridazine, pyridine, pyrazole, imidazole, oxazole, oxadiazole, thiazole, benzimidazole, benzofuran, benzothiophene, indole, dibenzofuran, dibenzothiophene, carbazole, indenocarbazole and indolocarbazole, where these groups may each be substituted by one or more radicals R¹; or **in that** Ar, if L stands for C(=O), is selected from the group consisting of phenyl, biphenyl, ortho-, meta- or para-terphenyl, ortho-, meta-, para- or branched quaterphenyl, 1-, 2-, 3- or 4-fluorenyl or 1-, 2-, 3- or 4-spirobifluorenyl, which may in each case be substituted by one or more radicals R¹.

8. Compound according to one or more of Claims 1 to 7, **characterised in that** Ar is selected from the structures of the formulae (Ar-1) to (Ar-24), 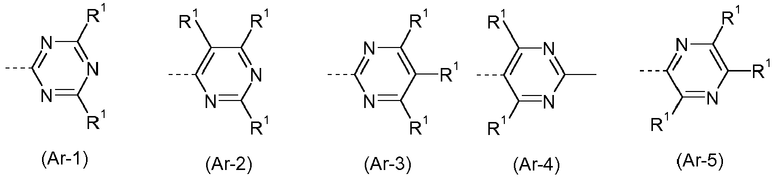 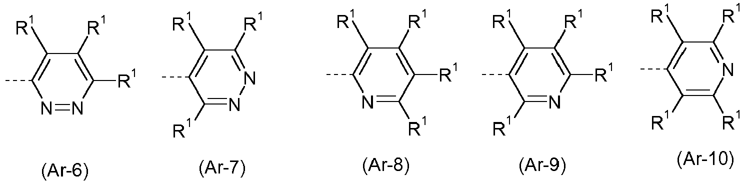 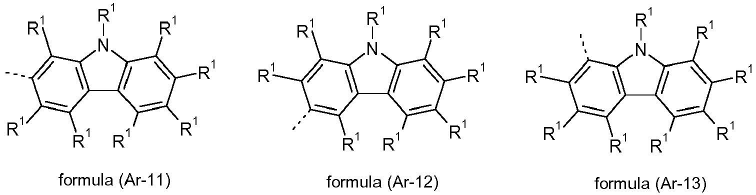 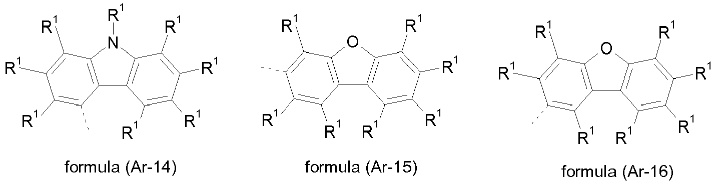 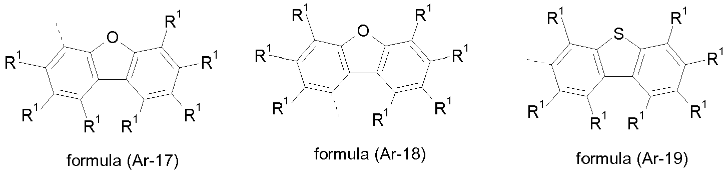 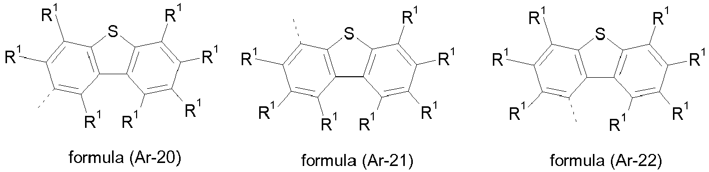 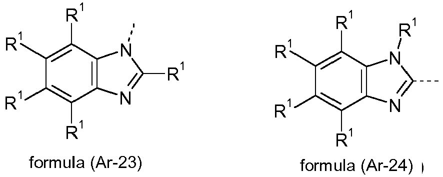 where the dashed bond indicates the bond to L and R¹ has the meanings given in Claim 1.

9. Compound according to one or more of Claims 1 to 8, **characterised in that** R and R¹ are selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, which may in each case be substituted by one or more radicals R², where one or more non-adjacent CH₂ groups may be replaced by O and where one or more H atoms may be replaced by F, or an aromatic or heteroaromatic ring system having 6 to 24 aromatic ring atoms, which may in each case be substituted by one or more radicals R².

10. Compound according to one or more of Claims 1 to 9, **characterised in that** the following applies to the symbols used:
L is a single bond, C(=O) or an aromatic ring system having 6 to 12 aromatic ring atoms, which may be substituted by one or more radicals R;
Ar is a heteroaromatic ring system having 5 to 13 aromatic ring atoms, which may be substituted by one or more radicals R¹, where Ar is bonded to L via a carbon atom if L stands for a single bond, or is bonded to L via a carbon or nitrogen atom if L is not equal to a single bond; or is an aromatic ring system having 6 to 24 aromatic ring atoms, which may be substituted by one or more radicals R¹, if L stands for C(=O);
R, R¹ are selected, identically or differently on each occurrence, from the group consisting of H, D, F, CN, a straight-chain alkyl or alkoxy group having 1 to 10 C atoms or a branched or cyclic alkyl or alkoxy group having 3 to 10 C atoms, which may in each case be substituted by one or more radicals R², where one or more non-adjacent CH₂ groups may be replaced by O and where one or more H atoms may be replaced by F, an aromatic or heteroaromatic ring system having 6 to 24 aromatic ring atoms, which may in each case be substituted by one or more radicals R².

11. Process for the preparation of a compound according to one or more of Claims 1 to 10, **characterised in that** the group -L-Ar is introduced by a metal-catalysed coupling reaction between a 1- or 4-functionalised spirobifluorene and a functionalised group -L-Ar.

12. Formulation comprising at least one compound according to one or more of Claims 1 to 10, in particular a solution, dispersion or miniemulsion comprising at least one compound according to one or more of Claims 1 to 10 and at least one solvent, or a mixture comprising at least one compound according to one or more of Claims 1 to 10 and at least one fluorescent or phosphorescent compound.

13. Use of a compound according to one or more of Claims 1 to 10 or a formulation according to Claim 12 in an electronic device, in particular in an organic electroluminescent device.

14. Electronic device comprising at least one compound according to one or more of Claims 1 to 10 or at least one formulation according to Claim 12, where the electronic device is preferably selected from the group consisting of organic electroluminescent devices, organic integrated circuits, organic field-effect transistors, organic thin-film transistors, organic light-emitting transistors, organic solar cells, dye-sensitised organic solar cells, organic optical detectors, organic photoreceptors, organic field-quench devices, light-emitting electrochemical cells, organic laser diodes and organic plasmon emitting devices.

15. Electronic device according to Claim 14, which is an organic electroluminescent device, **characterised in that** the compound according to one or more of Claims 1 to 10 is employed as electron-transport material in an electron-transport or hole-blocking layer or as matrix material for fluorescent or phosphorescent emitters in an emitting layer.

## Revendications

1. Composé de la formule (1) ou (2) : 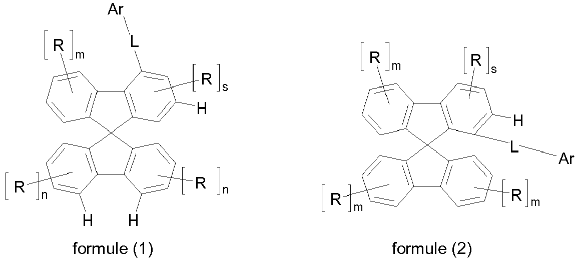 dans lesquelles ce qui suit s'applique aux symboles et indices qui sont utilisés :
Ar est un système de cycle hétéroaromatique qui comporte 5 à 40 atomes de cycle aromatique, lequel est lié à L via un atome de carbone si L représente une liaison simple, et lequel est lié à L via un atome de carbone ou un atome d'azote si L n'est pas égal à une liaison simple, et lequel peut être substitué par un radical ou par plusieurs radicaux R¹ ; ou Ar est un système de cycle hétéroaromatique qui comporte 6 à 40 atomes de cycle aromatique, lequel peut être substitué par un radical ou par plusieurs radicaux R¹, si L représente C(=O) ;
L est une liaison simple, C(=O) ou un système de cycle qui comporte 5 à 24 atomes de cycle aromatique, lequel peut être substitué par un radical ou par plusieurs radicaux R ;
R, R¹ sont sélectionnés pour chaque occurrence, de manière identique ou différente, parmi le groupe qui est constitué par H, D, F, Cl, Br, I, CN, Si(R²)₃, un groupe alkyle, alcoxy ou thioalkyle en chaîne droite qui comporte 1 à 40 atome(s) de C ou un groupe alkyle, alcoxy ou thioalkyle ramifié ou cyclique qui comporte 3 à 40 atomes de C, lequel peut dans chaque cas être substitué par un radical ou par plusieurs radicaux R², où dans chaque cas un ou plusieurs groupe(s) CH₂ non adjacents peut/peuvent être remplacé(s) par Si(R²)₂, C=NR², P(=O)(R²), SO, SO₂, NR², O, S ou CONR² et où un ou plusieurs atome(s) de H peut/peuvent être remplacé(s) par D, F, Cl, Br ou I, un système de cycle aromatique ou hétéroaromatique qui comporte 6 à 40 atomes de C, lequel peut dans chaque cas être substitué par un radical ou par plusieurs radicaux R², un groupe aryloxy qui comporte 5 à 60 atomes de cycle aromatique, lequel peut être substitué par un radical ou par plusieurs radicaux R², ou un groupe aralkyle qui comporte 5 à 60 atomes de cycle aromatique, lequel peut dans chaque cas être substitué par un radical ou par plusieurs radicaux R², ou deux substituants R ou R¹ adjacents ou plus peuvent en option former un système de cycle aliphatique, monocyclique ou polycyclique, lequel peut être substitué par un radical ou par plusieurs radicaux R²
R² est sélectionné parmi le groupe qui est constitué par H, D, F, un radical hydrocarbone aliphatique qui comporte 1 à 20 atome(s) de C ou un système de cycle aromatique ou hétéroaromatique qui comporte 5 à 30 atomes de C, dans lequel un ou plusieurs atome(s) de H peut/peuvent être remplacé(s) par D ou F, où deux substituants R² adjacents ou plus peuvent former un système de cycle aliphatique, monocyclique ou polycyclique l'un avec l'autre ou les uns avec les autres ;
s est 0, 1 ou 2 ;
m est pour chaque occurrence, de manière identique ou différente, 0, 1, 2, 3 ou 4;
n est pour chaque occurrence, de manière identique ou différente, 0, 1, 2 ou 3.

2. Composé selon la revendication 1, sélectionné parmi les composés de la formule (1a) et de la formule (2a) : 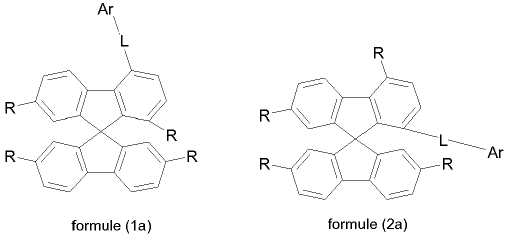 dans lesquelles les symboles qui sont utilisés présentent les significations qui ont été données selon la revendication 1.

3. Composé selon la revendication 1 ou 2, sélectionné parmi les composés des formules (1b) et (2b) : 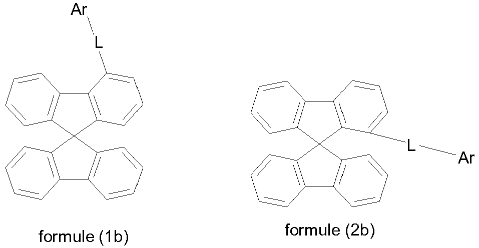 dans lesquelles les symboles qui sont utilisés présentent les significations qui ont été données selon la revendication 1.

4. Composé selon une ou plusieurs des revendications 1 à 3, **caractérisé en ce que** L est sélectionné parmi une liaison simple, C(=O) ou un système de cycle aromatique qui comporte 6 à 12 atomes de cycle aromatique, lequel peut être substitué par un radical ou par plusieurs radicaux R.

5. Composé selon une ou plusieurs des revendications 1 à 4, **caractérisé en ce que** L est sélectionné parmi le groupe qui est constitué par une liaison simple ou un groupe phénylène ortho-lié, méta-lié ou para-lié, lequel peut être substitué par un radical ou par plusieurs radicaux R, mais est de préférence non substitué.

6. Composé selon une ou plusieurs des revendications 1 à 5, **caractérisé en ce que** Ar représente un système de cycle hétéroaromatique qui comporte 5 à 24 atomes de cycle aromatique, en particulier qui comporte 5 à 13 atomes de cycle aromatique, lequel peut dans chaque cas être substitué par un radical ou par plusieurs radicaux R¹ ; ou **en ce que** Ar représente un système de cycle aromatique qui comporte 6 à 24 atomes de cycle aromatique, lequel peut être substitué par un radical ou par plusieurs radicaux R¹, si L représente C(=O).

7. Composé selon une ou plusieurs des revendications 1 à 6, **caractérisé en ce que** Ar est sélectionné parmi le groupe qui est constitué par triazine, pyrimidine, pyrazine, pyridazine, pyridine, pyrazole, imidazole, oxazole, oxadiazole, thiazole, benzimidazole, benzofurane, benzothiophène, indole, dibenzofurane, dibenzothiophène, carbazole, indénocarbazole et indolocarbazole, où ces groupes peuvent chacun être substitués par un radical ou par plusieurs radicaux R¹ ; ou **en ce que** Ar, si L représente C(=O), est sélectionné parmi le groupe qui est constitué par phényle, biphényle, ortho-terphényle, méta-terphényle ou para-terphényle, ortho-quaterphényle, méta-quaterphényle, para-quaterphényle ou quaterphényle ramifié, 1-, 2-, 3- ou 4-fluorényle ou 1-, 2-, 3- ou 4-spirobifluorényle, lequel peut dans chaque cas être substitué par un radical ou par plusieurs radicaux R¹.

8. Composé selon une ou plusieurs des revendications 1 à 7, **caractérisé en ce que** Ar est sélectionné parmi les structures des formules (Ar-1) à (Ar-24) : 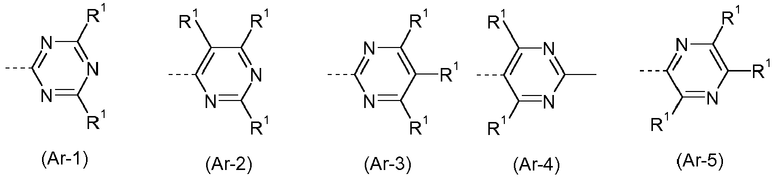 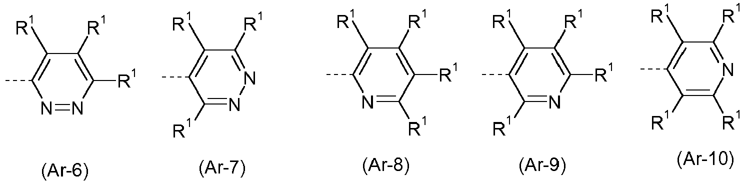 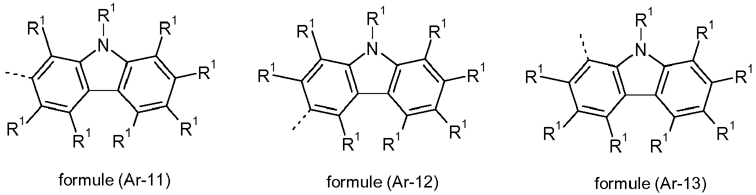 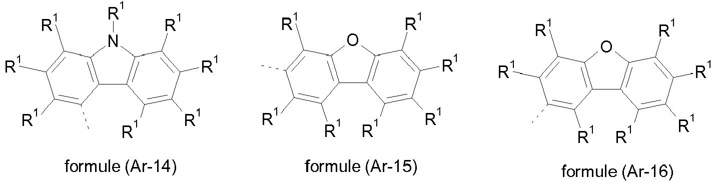 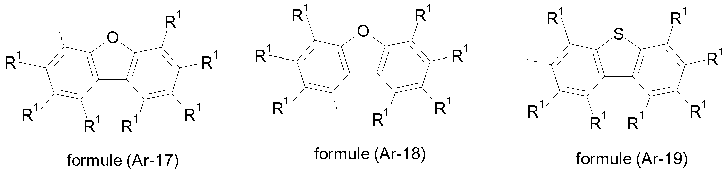 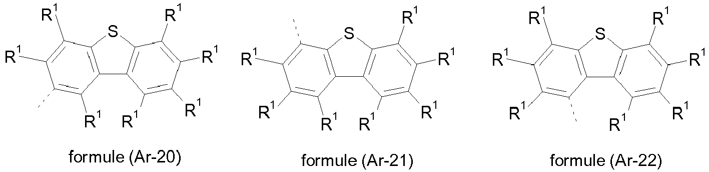 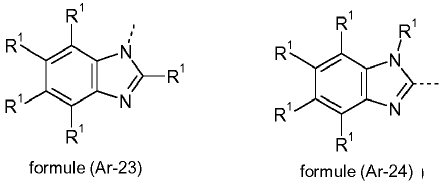 dans lesquelles le lien en pointillés indique le lien sur L et R¹ présente les significations qui ont été données selon la revendication 1.

9. Composé selon une ou plusieurs des revendications 1 à 8, **caractérisé en ce que** R et R¹ sont sélectionnés, de manière identique ou différente pour chaque occurrence, parmi le groupe qui est constitué par H, D, F, CN, un groupe alkyle ou alcoxy en chaîne droite qui comporte 1 à 10 atome(s) de C ou un groupe alkyle ou alcoxy ramifié ou cyclique qui comporte 3 à 10 atomes de C, lequel peut dans chaque cas être substitué par un radical ou par plusieurs radicaux R², où un ou plusieurs groupe(s) CH₂ non adjacents peut/peuvent être remplacé(s) par O et où un ou plusieurs atome(s) de H peut/peuvent être remplacé(s) par F, ou un système de cycle aromatique ou hétéroaromatique qui comporte 6 à 24 atomes de cycle aromatique, lequel peut dans chaque cas être substitué par un radical ou par plusieurs radicaux R².

10. Composé selon une ou plusieurs des revendications 1 à 9, **caractérisé en ce que** ce qui suit s'applique aux symboles qui sont utilisés :
L est une liaison simple, C(=O) ou un système de cycle aromatique qui comporte 6 à 12 atomes de cycle aromatique, lequel peut être substitué par un radical ou par plusieurs radicaux R ;
Ar est un système de cycle hétéroaromatique qui comporte 5 à 13 atomes de cycle aromatique, lequel système de cycle peut être substitué par un radical ou par plusieurs radicaux R¹, où Ar est lié à L via un atome de carbone si L représente une liaison simple, ou est lié à L via un atome de carbone ou un atome d'azote si L n'est pas égal à une liaison simple ; ou un système de cycle aromatique qui comporte 6 à 24 atomes de cycle aromatique, lequel système de cycle peut être substitué par un radical ou par plusieurs radicaux R¹, si L représente C(=O) ;
R, R¹ sont sélectionnés, de manière identique ou différente pour chaque occurrence, parmi le groupe qui est constitué par H, D, F, CN, un groupe alkyle ou alcoxy en chaîne droite qui comporte 1 à 10 atome(s) de C ou un groupe alkyle ou alcoxy ramifié ou cyclique qui comporte 3 à 10 atomes de C, lequel peut dans chaque cas être substitué par un radical ou par plusieurs radicaux R², où un ou plusieurs groupe(s) CH₂ non adjacents peut/peuvent être remplacé(s) par O et où un ou plusieurs atome(s) de H peut/peuvent être remplacé(s) par F, un système de cycle aromatique ou hétéroaromatique qui comporte 6 à 24 atomes de cycle aromatique, lequel peut dans chaque cas être substitué par un radical ou par plusieurs radicaux R².

11. Procédé pour la préparation d'un composé selon une ou plusieurs des revendications 1 à 10, **caractérisé en ce que** le groupe -L-Ar est introduit au moyen d'une réaction de couplage catalysée par métal entre un spirobifluorène 1-fonctionnalisé ou 4-fonctionalisé et un groupe -L-Ar fonctionnalisé.

12. Formulation comprenant au moins un composé selon une ou plusieurs des revendications 1 à 10, en particulier une solution, une dispersion ou une mini-émulsion comprenant au moins un composé selon une ou plusieurs des revendications 1 à 10 et au moins un solvant, ou mélange comprenant au moins un composé selon une ou plusieurs des revendications 1 à 10 et au moins un composé fluorescent ou phosphorescent.

13. Utilisation d'un composé selon une ou plusieurs des revendications 1 à 10 ou d'une formulation selon la revendication 12 dans un dispositif électronique, en particulier dans un dispositif électroluminescent organique.

14. Dispositif électronique comprenant au moins un composé selon une ou plusieurs des revendications 1 à 10 ou au moins une formulation selon la revendication 12, où le dispositif électronique est de préférence sélectionné parmi le groupe qui est constitué par les dispositifs électroluminescents organiques, les circuits intégrés organiques, les transistors à effet de champ organiques, les transistors à film mince organiques, les transistors à émission de lumière organiques, les cellules solaires organiques, les cellules solaires organiques sensibilisées par colorant(s), les détecteurs optiques organiques, les photorécepteurs organiques, les dispositifs à extinction de champ organiques, les cellules électrochimiques à émission de lumière, les diodes laser organiques et les dispositifs à émission de plasmons organiques.

15. Dispositif électronique selon la revendication 14, lequel est un dispositif électroluminescent organique, **caractérisé en ce que** le composé selon une ou plusieurs des revendications 1 à 10 est utilisé en tant que matériau de transport d'électrons dans une couche de transport d'électrons ou de blocage de trous ou en tant que matériau de matrice pour des émetteurs fluorescents ou phosphorescents dans une couche d'émission.
